# EUROPEAN PATENT SPECIFICATION

(11) **EP 1 399 192 B1**
(45) Date of publication and mention of the grant of the patent: **07.11.2007**
(21) Application number: 02748760.2
(22) Date of filing: 11.06.2002
(51) Int. Cl.: A61K 47/48

(54) **CONJUGATES OF POLYSACCHARIDE POLYMERS OF NATURAL ORIGIN**
KONJUGATE VON POLYMEREN NATÜRLICHEN URSPRUNGS
CONJUGUES DE POLYMERES DE POLYSACCHARIDE D'ORIGINE NATURELLE

(30) Priority: 12.06.2001 IT MI20011238
(43) Date of publication of application: 24.03.2004
(73) Proprietor: Bartholdy-Consultadoria e Servicos Lda, Funchal (PT)
(72) Inventor: VOLPATO, Ivo, I-06070 S. Mariano (IT); BIZZINI, Bernard, Emile, F-81000 Albi (FR); ABREU,Roberto,Carlos,c/o Bartholdy-Consultadoria, 6° Andar,Sala 605 P-9000 FUNCHAL MADEIRA (PT); LIPPMANN, Marco, 6957 Roveredo/Ti (CH)
(74) Representative: Gervasi, Gemma
(86) International application number: PCT/EP2002/006371
(87) International publication number: WO 2002/100440

(56) References cited:
- EP-A- 0 611 573
- WO-A-90/15628
- WO-A-93/11803
- WO-A-97/38312
- DE-A- 4 029 374
- GB-A- 816 750
- GB-A- 900 803
- KALPAXIS, D. L. ET AL: "Immobilization of hyaluronate on cellulose fibers and its use for the isolation of cartilage components" INT. J. BIOCHEM. (1985), 17(1), 61-6, XP001041420
- DATABASE WPI Section Ch, Week 199123 Derwent Publications Ltd., London, GB; Class A17, AN 1991-168375 XP002183306 & JP 03 101618 A (KURARAY CO LTD), 26 April 1991 (1991-04-26)
- EDWARDS, J. V.: "Synthesis and formulation of covalently and ionically bound peptido- cellulose conjugates" BOOK OF ABSTRACTS, 215TH ACS NATIONAL MEETING, DALLAS, MARCH 29-APRIL 2 (1998), CELL-047 PUBLISHER: AMERICAN CHEMICAL SOCIETY, WASHINGTON, D. C. , XP001112977

## Description

### FIELD OF THE INVENTION

The present invention relates to the use of fibres of polysaccharide polymers of natural origin, preferably of vegetal origin, such as for instance cellulose or cotton, or the use of yarns, non-woven fabrics (or felts), or fabrics obtained from said fibres in order to obtain pharmaceutical, cosmetic or hygienic products, household articles or products to be used in food industry. In particular, the polysaccharide polymers according to the invention can be used to obtain plasters, gauzes, sanitary cotton wool, vaginal and surgical tampons, bandages, gloves, stockings, masks, curtains, carpets and the like. Moreover, the polysaccharide polymers according to the invention can be used to obtain fabrics, filters or wrappings to be used in the field of food industry.

### STATE OF THE ART

Fibres of polysaccharide polymers of natural origin, preferably of vegetal origin, such as for instance cellulose or cotton, or yarns, non-woven fabrics (or felts), or fabrics obtained from said fibres are used in the medical field, for self-medication or in the field of hygienic or cosmetic products with merely mechanical or supporting functions. The same can be said for the field of food industry, where cotton fabrics are used for instance to filter cheese or in the production of sausages (salamis). Moreover, fibres of polysaccharide polymers of natural origin, prefrably of vegetal origin, are used for the manufacture of household articles like carpets, curtains or the like.

In general, in the medical field, pharmacologically or biologically active substances in form of powders, creams or ointments, are applied onto the body, in particular on the tissue site to be treated, then the cotton support is applied as a mere covering and protecting means.

Sometimes, when the medicinal substance is soluble, as in the case of disinfectants, said substance is sprayed onto the cotton wool which is then rubbed onto the skin for the application.

On the other hand, in the field of food industry the fibres or yarns and the like which are used are previously sterilized by means of disinfectants or treatment with vapor, UV rays, etc.

Likewise, household articles like carpets and curtains are subjected from time to time laundering or dry-cleaning for removing dust and possibly also mite populations possibly harboured therein.

However, in some cases these procedures are no good remedy.

That is the case for instance:
a) Of sores, bedsores, wounds:
   The pharmaceuticals which are applied in these cases (anti-bacterial agents, anti-inflammatory agents, antibiotics, etc.) generally consist of cold creams, gels, ointments, powders or sprays, i.e. not only of the pure active principle, but of pharmaceutical compositions (comprising excipients such as e.g. adjuvants, preserving agents and the like) which are optimized for the desired application onto the site to be treated.
   Despite their indubitable simplicity of application, it is broadly known that for instance creams, gels and ointments, because of the often reduced bioavailability of the active principles they contain, delay cicatrisation, whereas powders can cause the formation of granulomas, and sprays do not always ensure the correct delivery of medicament to the wounded site. Therefore, the use of pharmaceutical excipients, though their choice is made aiming at the maximum compatibility with the patient's tissues, can involve evident disadvantages.
b) Of disinfection of skin areas:
   The disinfectants, generally soluble, which are used to imbibe cotton wools, do not stay for a long time in high amounts on the site of application, they are withdrawn by washing off and do not therefore ensure a good effectiveness.
c) In all cases when gauzes or bandages used to protect injured and wounded areas, skin inflammations and erosions, tend to accumulate powder and, potentially, infected material with time, thus representing a substantial risk if not frequently replaced or if not handled correctly (in sterile conditions).
d) Also of protecting products such as gloves and the like which might share the same problem; for example, they can accumulate allergizing, infected material or other.
e) Of vaginal tampons for menstruations which might have a partial effectiveness because of the imbibition with erythrocytes and platelets on the outer surface, which might prevent the absorption by the inner layers.
f) In the use of sanitary towels, where the same problem may arise.
g) Also of yarns or fabrics used in food industry, for instance to filter cheese, but also to prepare sausages, where the same problem may arise; for instance, in spite of initial sterilization they can then accumulate non-sterile material.
i) Finally, as well for household articles, which must be cleaned with high frequency to keep mite contamination suitably low, which is rather uncomfortable and laborious for certain household articles like e.g. materasses.

An object of the present invention is therefore to provide for pharmaceutical (in particular drugs and medical items), hygienic or cosmetic products which do not show the aforesaid disadvantages and which ensure the optimal delivery of active principle to the injured site. Such pharmaceutical (in particular drugs and medical items), hygienic or cosmetic products could therefore lead not only to a high reduction in the use of the active principle, but above all to a considerable increase in the effectiveness of the treatment.

In particular, a further object of the present invention is to provide for plasters, gauzes, cotton wool, bandages and the like which can be applied directly onto the site to be treated and which do not contain the usual and potentially disadvantageous pharmaceutical excipients contained in the compositions as described above.

Another object of the present invention is to provide for plasters, gauzes, cotton wool, bandages and the like which can be applied directly onto the site to be treated without any phenomenon of washing off of the active agent.

An additional object of the present invention is to provide for plasters, gauzes, cotton wool, bandages and the like which can be applied directly onto the site to be treated and which are not susceptible to the accumulation of infective or allergenic material.

A further object of the present invention is to provide for surgical tampons with high effectiveness.

Another object of the present invention is to provide for hygienic items such as for instance vaginal tampons or sanitary towels with high absorbing power.

An additional object of the present invention is to provide for cosmetic items whose application onto the skin is not difficult and/or does not cause any discomfort.

A further object of the, present invention is to provide for articles of clothing of antiallergenic yarns.

Another object of the present invention is to provide for yarns, fabrics, non-woven fabrics and the like suitable for use in food industry, which are not susceptible to the accumulation of non-sterile material, thus contributing to a long-term preservation of the food obtained through their use.

Another object of the present invention is to provide for household articles made from textiles or non-woven fabrics which are non-susceptible to the contamination with mites.

WO 93/11803 relates to a non-woven fabric comprising hyaluronic acid and cellulose. The modified non-woven fabric finds its application in therapeutic methods.

Kalpexis et al. (1985) Int. J. Biochem. 17, 61-66 discloses the covalent immobilization of hyaluronate on cellulose fibers using the linker ε-amino-n-caproic acid. The modified cellulose was used to isolate proteoglycans.

Edwards et al. (2000) Bioconjugate Chem. 11, 469-473 discloses the conjugates between cotton cellulose and peptides to prepare textiles which can be used for instance in wound healing. In particular, a lysozyme was coupled to glycine derivatized cotton cellulose.

However, there remains the problem to be solved of how to improve the activity of the active agent.

### SUMMARY

These and other objects which will be apparent in the following are reached by a conjugate comprising a polysaccharide polymer of natural origin chemically bound to a pharmaco-logically or biologically active substance through a polylinker. In particular, fibres consisting of polysaccharide polymers according to the invention can be used to obtain non-woven fabrics (or felts) or yarns or fabrics which can be used in the manufacture of drugs, of medicinal, cosmetic or hygienic items, or in the manufacture of filters, fabrics or wrappings used in the field of food industry, as Well as in the manufacture of household articles. The chemical conjugation between polysaccharide polymers of natural origin, preferably of vegetal origin, and pharmacologically or biologically active substances can take place during any stage of manufacture, i.e. using as substrate exclusively the said natural fibres or optionally the non-woven fabrics, yarns or fabrics obtained downstream from said fibres.

Another aspect of the present invention is the provision of a method to obtain a polysaccharide polymer according to the invention or fibres, yarns, fabrics or non-woven fabrics according to the invention, comprising the following steps:
(a) the creation, on the fibres of polysaccharide polymer or on the yarns, fabrics or non-woven fabrics obtained from said fibres, of sites which are suitable for the chemical conjugation, thus obtaining an activated polysaccharide polymer, and
(b) the reaction between the activated polysaccharide polymer and a pharmacologically or biologically active substance or of a derivative thereof under formation of a chemical conjugation between the activated polysaccharide polymer and the pharmacologically or biologically active substance, and optionally
(c) the modification, by means of chemical reaction, of the nature of the conjugation established in steps (a) and (b) between the polysaccharide polymer and the pharmacologically or biologically active substance.

### DETAILED DESCRIPTION OF THE INVENTION

The polysaccharide polymers according to the invention consist of polysaccharide polymers of natural origin, chemically conjugated with pharmacologically or biologically active substances. Polysaccharide polymers of vegetal origin are particularly preferred.

The polysaccharide polymer of natural origin used in the present invention should be substantially non-biodegradable at the site of therapeutic application (or for its use in food industry) and be preferably easily available, cheap, easy to be sterilized, non-susceptible to depolymerization when undergoing reactions of chemical conjugation, and it should above all form macroscopic fibres which are easy to be worked. A preferred example for a polysaccharide polymer of natural origin is a polysaccharide polymer of vegetal origin, for instance cellulose, i.e. poly-D-glucose in which the units of D-glucose are joined by means of β-glucoside bonds between the anomer carbon of a molecule and the OH group of the C-4 of another molecule. A particularly preferred form of cellulose for the present invention is cotton. Another preferred form of cellulose according to the invention is the so-called "regenerated cellulose" or viscose.

The pharmacologically or biologically active substances according to the present invention to be conjugated with the polysaccharide polymer of natural origin can have different chemical nature and structure on the basis of the functional or exogenous biochemical parameters which are involved in the pathology and which have to be neutralized or rebalanced in order to obtain a given prophylactic or therapeutic effect. As a mere example and without excluding further therapeutic or prophylactic properties, the pharmacologically or biologically active substances can show anti-bacterial, anti-inflammatory, antibiotic, anti-mycotic, fungistatic, anti-mite, disinfectant, anti-hemorrhagic, antiseptic, immunostimulating, anti-traumatic, anti-allergic, cicatrizing, riepithelizing, anti-erythematic, anti-dermatopathic, anti-burn, anesthetic, anti-coagulating, coagulating, anti-varix, anti-phlebitis, anti-aggregating for platelets, fibrinolytic, lipolytic, wrinkle-preventing or anti-aging properties.

As far as the nature of the pharmacologically or biologically active substance is concerned, the only limitation imposed by the present invention is that it can be chemically conjugated with a polysaccharide polymer of natural origin.

Among pharmacologically or biologically active substances as indicated above, those characterized by the presence of at least an amino, carboxyl, thiol or carbonyl group are particularly preferred.

Known pharmacologically or biologically active substances, though without amino, carboxyl, thiol or carbonyl groups, can also be used in the preferred embodiment of the invention as indicated above, if said substances can be derivatized by introducing one of these groups (for instance by oxidizing an alcohol hydroxyl group to obtain a carbonyl or carboxyl group) without any loss or any substantial reduction of the pharmacological or biological activity, i.e. to obtain a further pharmacologically or biologically active substance. Preferred though not exclusive examples of pharmacologically or biologically active substances which can be conjugated directly or after derivatization with polysaccharide polymers of natural origin, preferably of vegetal origin, are the following: hexachlorocyclohexane, benzylbenzoate, mesulfene, 4-hydroxybenzaldehyde, sulfadiazine, sulfadimethoxine, bacitracin, gramicidin, ketanserine, procaine, nistatin, epidermal growth factor (EGF), dermatan sulfate (condroitin sulfate B - Sigma C 4259), F VIII (anti-hemophilic plasmatic factor), proteins and immunostimulating glycoproteins, fibrinogen or its degradation products, the so-called "FDP", heparin (preferably of low molecular weight), prothrombin, naproxen, diclofenac, lipase, immunoglobulins (lgG), hyaluronic acid, collagenase, corticosteroids or elastin. Among corticosteroids cortisol ("hydrocortisone") is particularly preferred. Among immunostimulating proteins the immunomodulating fraction from Corynebacterium granulosum is particularly preferred. Preferred though not exclusive examples of pharmacologically or biologically active substances conjugated with polysaccharide polymers of vegetal origin can be found in the experimental parts of the present application.

The chemical conjugation according to the invention consists in the insertion - between one previously activated site of the polysaccharide polymer and the pharmacologically or biologically active substance - of a chain of several linkers bound in turn one to another, the first end of the chain of linkers being bound to a site on the polysaccharide polymer and the second end of the chain of linkers being bound to the pharmacologically or biologically active substance.

In a particularly preferred embodiment of the invention said chemical conjugation between the polysaccharide polymer and the pharmacologically or biologically active substance comprises trivalent linkers enabling the conjugation of a site on the polysaccharide polymer with two molecules of pharmacologically or biologically active substance.

The term linker as used in the present invention comprises both, single bivalent atoms such as for instance -S-, and whole at least bivalent groups, such as for instance the following lysyl fragment:

-NH-CH₂(CH₂)₃-CH(COOH)-NH-

which is introduced into the polysaccharide polymers according to the invention through the reaction with the amino acid lysine. Further preferred linkers according to the present invention are aspartic acid and glutamic acid. Another preferred linker according to the present invention is cysteine. An additional preferred linker according to the present invention is thio-threonine, that is to say a threonine whose OH group has been replaced with a SH group. According to the specific needs, in some preferred cases lysine and cysteine (as well as aspartic acid, glutamic acid and also thio-threonine) can also be trivalent linkers. For the sake of terminological simplicity, in the framework of the present invention, the term "linker" refers both, to the substance from which the inserted atom or fragment (e.g. "H₂S" in the case of -S-) formally derives or the reagent which is used to establish or modify a chemical conjugation (e.g. lysine), and to the bi- or multivalent fragment which is then part of the chemical conjugation thus obtained (e.g. the bivalent lysyl fragment as indicated above). Likewise, the term "linker" is intended to comprise -in the case of asymmetric linkers- racemic mixtures as well as the pure enantiomers.

The nature of the chemical bonds which are present in the conjugation between the polysaccharide polymer and the pharmacologically or biologically active substance (including - beyond those which are established during the formation of the chemical conjugation - also those which are already present within the linkers) can be different whether the pharmacologically or biologically active conjugated substance, in order to express its effectiveness, has to be released or may remain linked to the polymer itself. If release is desired, a chemical conjugation is chosen which is biodegradable in contact with the patient's body, i.e. a chemical conjugation containing at least a bond which is susceptible to cleavage under conditions of use. On the other hand, when the said substance's release is not desirable, a chemical conjugation is chosen which is not biodegradable in contact with the patient's body, i.e. a chemical conjugation whose bonds are stable under conditions of use.

In a particularly preferred embodiment of the invention, the chemical conjugation between the polysaccharide polymer and the pharmacologically or biologically active substance comprises one or more of the following bonds independently chosen from the group consisting of the imino bond, the secondary amino bond, the single bond between two nitrogen atoms, the double bond between two nitrogen atoms, the peptide bond and the disulfuric bond. The relative biodegradability of these bonds depends on the environment to which they are exposed, for instance on the presence of enzymes, reducing agents, etc. For instance, in the framework of the present invention, it has been found that the secondary amino bond is substantially non-biodegradable in contact with skin.

In a particularly preferred embodiment of the invention the chemical conjugation between the polysaccharide polymer and the pharmacologically or biologically active substance comprises a cysteinyl or lysyl linker, i.e. obtained from cysteine or lysine.

According to the invention, the chemical conjugation between the polysaccharide polymer and the pharmacologically or biologically active substance comprises a poly linker, in particular a poly lysine linker having preferably a molecular weight of less than 16000, eg. 4000-15000 or 1000-4000, the latter one being the most preferred.

In a particularly preferred embodiment of the invention, the chemical conjugation between the polysaccharide polymer and the pharmacologically or biologically active substance comprises a poly linker consisting of a cysteyl linker in turn linked to a polylysine linker having preferably a molecular weight of less than 16000, e.g. 4000-15000 or 1000-4000 the latter one being most preferred.

In a particularly preferred embodiment of the invention, the chemical conjugation between the polysaccharide polymer and the pharmacologically or biologically active substance comprises a linker obtained from glutamic or aspartic acid.

In a particularly preferred embodiment of the invention, the chemical conjugation between the polysaccharide polymer and the pharmacologically or biologically active substance comprises a poly linker, in particular a poly glutamic or aspartic acid linker having a molecular weight of less than 16000.

In a particularly preferred embodiment of the invention, the chemical conjugation between the polysaccharide polymer and the pharmacologically or biologically active substance comprises a poly linker consisting of a cysteyl linker in turn linked to a polyglutamic or aspartic acid linker having preferably a molecular weight of less than 16000.

In a particularly preferred embodiment of the invention, the chemical conjugation between the polysaccharide polymer and the pharmacologically or biologically active substance comprises a linker obtained from thio-threonine.

In a preferred embodiment of the invention the chemical conjugation between the pharmacologically or biologically active substance (or of a derivative thereof) and the polysaccharide polymer is located on a carbon atom of the polysaccharide polymer carrying (before the conjugation) a primary or secondary alcohol OH group.

In a further preferred embodiment, the chemical conjugation between the pharmacologically or biologically active substance (or of a derivative thereof) and the polysaccharide polymer is located on a carbon atom of the polysaccharide polymer carrying (before the conjugation) a secondary alcohol OH group.

As far as the synthesis of conjugation between the polysaccharide polymer of natural origin, preferably of vegetal origin, and the pharmacologically active substance is concerned, the same comprises
(a) the creation, on the polysaccharide polymer, of sites which are suitable for the chemical conjugation, thus obtaining an activated polysaccharide polymer, and
(b) the reaction between the activated polysaccharide polymer and a pharmacologically or biologically active substance or of a derivative thereof under formation of a chemical conjugation between the activated polysaccharide polymer and the pharmacologically or biologically active substance, and optionally
(c) the modification, by means of a chemical reaction, of the nature of the chemical conjugation established in steps (a) and (b) between the polysaccharide polymer and the pharmacologically or biologically active substance.

According to a preferred embodiment of the invention, the creation of sites suitable for the chemical conjugation on the polysaccharide polymer takes place by modifying at least some of the alcohol OH groups which are present on the polysaccharide polymer, thus obtaining an activated polysaccharide polymer. Preferred examples of activated polysaccharide polymers are polysaccharide polymers in which at least some of the carbon atoms of the polysaccharide polymer initially carrying primary or secondary alcohol OH groups are modified - directly on the aforesaid carbons or after introducing intermediate tinkers - so as to enable the formation of a chemical bond with a further linker or with a leaving group.

A leaving group is a group which can be replaced with another group in a following reaction.

With reference to step (c) as indicated above, modifications are preferred which can modulate (i.e. increase or decrease) the biodegradability of the chemical conjugation, such as for instance the reduction of an amino bond of a Schiff base in order to obtain the corresponding secondary amine. Another preferred method of synthesis according to the invention provides that step (a) as indicated above comprises in turn the following steps:
(a1) the oxidation or the substitution of at least some of the alcohol OH groups which are present on the polysaccharide polymer, thus obtaining a first activated polysaccharide polymer, and
(a2) the reaction between said first activated polysaccharide polymer and a first linker or a first leaving group, thus obtaining a second activated polysaccharide polymer, and
(a3) the modification, by means of a chemical reaction, of the nature of the chemical conjugation established in steps (a1) and (a2) between the first activated polysaccharide polymer and the linker or between the first activated polysaccharide polymer and the leaving group, thus obtaining a third activated polysaccharide polymer, and
(a4) the reaction between the second or third activated polysaccharide polymer, respectively, obtained in step (a2) or (a3) respectively, and a second linker or a second leaving group, thus obtaining a third or fourth activated polysaccharide polymer, respectively, in which the first linker has been extended with the second linker or in which the leaving group has been replaced by the second leaving group or by the second linker, and optionally
(a4+n) further repetitions of step (a4) to obtain other activated polysaccharide polymers.

With reference to the above step (a1), the oxidation can consist in the creation of aldehyde and/or ketone groups from the primary and/or secondary alcohol OH groups which are present on the polysaccharide polymer. At any rate, it is important that the conditions chosen are mild enough not to destroy the polymer chain. In a preferred embodiment, the oxidation is carried out with metaperiodate and involves the stereo-selective oxidation of the secondary alcohol OH groups forming the vicinal diol on the glucose ring.

With reference to the above step (a3), modifications are preferred which can modulate (i.e. increase or decrease) the biodegradability of the chemical conjugation, such as for instance the reduction of an amino bond of a Schiff base in order to obtain the corresponding secondary amine.

Another preferred method of synthesis according to the invention provides that step (a) as indicated above comprises in its turn the following stages:
(a1) the esterification of the alcohol OH groups which are present on the polysaccharide polymer with glutamic acid or aspartic acid, thus obtaining a first activated polysaccharide polymer, and optionally
(a2) the reaction between said first activated polysaccharide polymer and a linker or a leaving group, thus obtaining a second activated polysaccharide polymer, and
(a2+n) further repetitions of step (a2) to obtain other activated polysaccharide polymers.

With reference to the above step (a1), according to the conditions/concentrations chosen said esterification can be partial or involve both, the primary alcohol OH group as well as the secondary ones, until a quantitative esterification is obtained. Specific examples of preferred methods of synthesis according to the invention are the following:

Method A comprising the following steps:
(a1) at least partial oxidation of the alcohol OH groups present on the polysaccharide polymer, thus obtaining a first activated polysaccharide polymer comprising aldehyde groups, and
(a2) the reaction between said first activated polysaccharide polymer comprising aldehyde groups and lysine, thus obtaining a second activated polysaccharide polymer representing the Schiff base formed between said first activated polysaccharide polymer and one of the amino groups of lysine, and optionally
(a3) reduction of said second activated polysaccharide polymer, thus obtaining the third activated polysaccharide polymer in which the imino group present in the Schiff base has been reduced to the corresponding secondary amine.

Whereas in method A as indicated above, lysine gets bound to the activated polysaccharide polymer according to two different orientations (i.e. through its α-amino or ε-amino group) generating a "mixed" product, in stage (a2) it is also possible to introduce a lysyl linker in which, for instance, the α-amino position is suitably protected so as to enable the reaction of the activated polysaccharide polymer only with the ε-amino position of the lysyl linker, thus generating, after deprotection, a homogeneous product.

The linker lysine is particularly preferred in the present invention since it can react not only with pharmacologically or biologically active substances comprising carbonyl groups (i.e. aldehyde or ketone groups), but - under the formation of peptide bonds - also with pharmacologically or biologically active substances having amino or carboxyl groups.

Following method A, the lysil linker is extended (for instance by addition of other amino acids, analogously to the synthesis of polypeptides on solid phase according to Merrifield) before establishing the bond (or the bonds) with the pharmacologically or biologically active substance, thereby building up a poly linker, in particular a poly lysyl linker, theoretically capable of receiving at least one equivalent of pharmacologically or biologically active substance per lysyl residue. In the alternative, the poly linker may be linked as such or through further linkers to the activated polysaccharide polymer. Thus, in case that the poly linkers is made from tri- (or higher) valent linkers (as e.g. in the case of polyglutamic or polyaspartic acid as well as in the case of the preferred poly lysysl linker) a considerable "leverage" with respect to the activated site on the polymer may be obtained in that several equivalents of pharmacologically or biologically active substance may be bound to the same site. With certain poly linkers like e.g. poly lysine, and depending on the conditions adopted, some crosslinking of the active sites on the polymer through the poly linker may take place to a minor extent ("curing effect). Thus both, such "leverage" and such "curing" allow for the substantial preservation of the polymer's mechanical properties compared to direct conjugation. Apart from the leverage effect, even if the pharmacologically or biologically active substance is conjugated only to the most remote linker residue of the poly linker, an increase in conjugation efficiency and an optimized activity may arise. The latter improvements seem to be due to a "spacer effect" provided by the extended poly linker chain providing for an increased motility of the pharmacologically or biologically active substance.

### Method B:

(a1) at least partial sulfonesterification of the alcohol OH groups present on the polysaccharide polymer by means of a reaction with tosyl chloride or mesyl chloride, thus obtaining a first activated polysaccharide polymer, and
(a2) the substitution of the tosyl or mesyl groups present on the first activated polysaccharide group with SH groups, thus obtaining a second activated polysaccharide polymer comprising thiol groups, and
(a3) reaction of the second activated polysaccharide polymer comprising thiol groups with cystine, thus obtaining a third activated polysaccharide polymer comprising cysteinyl residues connected to the second activated polysaccharide polymer through disulfur bonds, and
(b) formation of a Schiff base between a carbonyl group present in a pharmacologically or biologically active substance or in one of its derivatives and the amino group of the cysteinyl residue.

The product obtained according to Method C(b) is an example of a substance according to the invention in which the chemical conjugation between the polysaccharide polymer and the pharmacologically or biologically active substance is established through two linkers, i.e. the linker -S- and the linker cysteine.

The linker cysteine is particularly preferred in the methods according to the present invention since, beyond the possibilities of formation of peptide bonds on its carboxyl and amino terminal, as described above for lysine, cysteine can also react with substances showing a thiol group as a leaving group as disclosed in the following:

According to a further preferred embodiment of the present invention thiol-threonine may replace cysteine as linker in some situations.

As a consequence, the extreme versatility of some of the methods of synthesis according to the invention has been shown, in particular of those particularly preferred methods involving the modification of the alcohol OH groups of the polysaccharide polymer together with the optional use of H₂S, cysteine or lysine as linkers or as leaving groups, for the conjugation of pharmacologically or biologically active substances having amino, thiol, carboxyl or carbonyl groups. In particular, it has been shown that the previous one is a series of syntheses of conjugation allowing to bind to the polysaccharide polymer, for instance the cotton or viscose fibre, drugs or biologically- active substances having a different nature and different pharmacological properties. It should also be observed that the series of syntheses of conjugation also enables - depending on the pharmacologically or biologically active substance used - a chemical conjugation which, as far the length (controllable by introducing linkers, for instance of amino acid nature) of the established connection, the eventual biodegradability of the bonds included in the chemical conjugation and the position of the connection on the polysaccharide polymer and on the pharmacologically or biologically active substance are concerned, is "tailored on demand". Further examples of preferred methods of synthesis according to the present invention are described in the experimental part of the present application.

It is important that the chemical conjugation can be carried out indifferently on the various macroscopic forms of the polysaccharide polymer, i.e. on the fibres or on the various worked products obtained from said polymer, e.g. on the cotton fibre, on the yarn (semi-finished product) or on the finished product (fabric, non-woven fabric, gauze, bandage) which can be used as such or to prepare other products such as tampons, gloves, masks, stockings, sanitary towels, etc. It should be noted that the possibility to carry out the chemical conjugation on the fibre or on the semi-finished product results in the possibility to provide for finished products supporting two or more pharmacologically or biologically active substances. For examples, by using two differently derivatized yarns, gauzes can be obtained comprising for instance both a disinfectant substance (bound for instance by non-biodegradable chemical conjugation) and a therapeutic substance undergoing release by biodegradation of the corresponding chemical conjugation. Said multiactive gauzes according to the invention are very advantageous because they allow to spare material with respect to traditional medications which would require the application of a disinfectant and of a medicament and the daily replacement of the gauze.

As far as the degree of conjugation with pharmacologically or biologically active substance is concerned, it can be varied by the man skilled in the art according to the various needs - by varying for instance reaction temperature and time or by using blocking agents (glycine for instance) - between a partial (under-stoichiometric) or substantially total. (stoichiometric) use of the sites which are suitable for the chemical conjugation on the polysaccharide polymer. The use of tri- or multifunctional linkers as poly linkers further increases the degree of total tri- or multifunctional linkers as poly linkers further increases the degree of total chemical conjugation which can be reached by the man skilled in the art according to the various needs.

As far as the use of polysaccharide polymers according to the invention in the preparation of drugs, and the drugs or medicinal, cosmetic or hygienic items thus obtained are concerned, you will now find the following examples representing preferred though not exclusive embodiments of the invention:

### 1. Vaginal tampons and sanitary towels:

### 1.a. Anti-hemorrhagics.

Polysaccharide polymer of vegetal origin, in particular cotton conjugated with prothrombin or fibrinogen or fibrin or coagulating Factor VIII. In this case the chemical bond can be stable, i.e. non-biodegradabte in contact with the patient. The conjugated coagulating active agents of proteic nature contribute to build an aggregate (platelets + fibrin), thus reducing the hemorrhagic effect during menstruations. Such a tampon is an example of a hygienic item according to the invention.

### 1.b. Anti-infection effect:

### a. Antimycotics.

The examples taken are the polysaccharide polymer of vegetal origin, in particular cotton conjugated with myconazol, nistatin and benzoic acid.

Myconazol is particularly active in vulvo-vaginal candidiasis, nistatin acts in particular on candidiasis, on infections caused by Trichophyton glabrata, by

Epidermophyton, etc.; benzoic acid, generally associated with salicylic, acid, is used as a general topical mycostatic.

### b. Antiseptics (disinfectant).

The example taken is cotton fibre conjugated with phenol, i.e. to 4-hydroxybenzaldehyde as phenol derivative.

Phenol and its derivatives develop their germicidal action with a mechanism of protein denaturation: they have a high penetrability into tissues and are therefore very toxic.

Their non-biodegradable conjugation with the cotton fibre during use prevents its absorption and therefore toxic effects.

### c. Anti-bacterials.

The examples taken are cotton fibre conjugated with bacitracin, gramicidin and sulfadiazine.

### 1.c. Anti-inflammatories:

The cotton fibre is conjugated with FANS anti-inflammatories (e.g. naproxen, diclofenac) and corticosteroids such as for instance cortisol.

### 1.d. Immunostimulating:

The stimulation of the functions of immunocompetent cells in the sub-mucous tissue can increase the defenses against pathogens and antagonize tissue phlogistic processes of different origin.

In this case the active agent to be conjugated with the cotton consists of glycoproteins with non-specific immunostimulating activity and of microorganic origin: for instance from Corynebacterium granulosum, parvum and catarrhalis (B. Bizzini, B. Maro and P. Lalouette, Médecine et Mal. Inf., 1978, 8, 408; T. Metianu e B. Bizzini, Comp. Immun. Microbiol. Infect. Dis., 1981, 4, 285).

### 2. Stockings and tights:

### 2.a. Anti-varix and anti-phlebities.

Varices and phlebitis involve the coagulating and parietal function of blood capillaries.

One of their causes seems to be due to the fact that the continuous contact between skin areas particularly full of blood vessels and fibres, in particular artificial fibres such as nylon, can reduce their contact with air and their subsequent oxygenation.

A good medicament to prevent and treat varicophlebitis should have anti-coagulating, anti-aggregating for platelets and fibrinolytic properties.

These properties belong to heparin and to heparin with low molecular weight (MW of about 6,000 D - Sigma H 2149).

Its conjugation with the cotton fibre can optimize its local effects by an in-situ permanence.

### 2.b. Anti-traumas.

In the treatment of skin traumas due to mechanical stimuli, radiations or other causes, gauzes or bandages in cotton fibre - derivatized both with heparin with low molecular weight and with steroid anti-inflammatories (example: cortisol) or with FANS - can be useful.

The same materials conjugated with non-specific immunomodulating glycoproteins can be used as successfully as in the previous case.

### 2.c. Anti-deposit of lipids.

In order to treat subcutaneous deposits and pads of fat it is possible to use stockings, tights, pants, shorts, bandages made with cotton fibre derivatized with the enzyme lipase (type Vll - Sigma L 1754), which are examples of the cosmetic items according to the invention.

The enzyme, maintained on the site in an adhering position, can penetrate as far as the sub-mucosa developing its lytic and mobilizing effect.

Improved results can be obtained by obtaining out the tissue with mixed fibres derivatized with lipase and with heparin with low molecular weight.

### 3. Anti-allergic gloves.

The basic idea to obtain gloves or other materials (e.g. gauzes) with an anti-allergic effect is to conjugate the cotton fibre with immunoglobulins which are specific against the allergen.

As a consequence of the long contact, the allergen itself can be complexed and therefore deactivated.

Better results can be obtained if the fabric is obtained with mixed fibres chosen among those which are derivatized with immunoglobulins and among those which are derivatized with immunostimulating glycoproteins.

### 4. Gauzes.

### 4.a. Disinfectants.

The examples mentioned above, with cotton fibre derivatized with germicides, antimycotic or anti-bacterial substances, are valid.

### 4.b. Cicatrizings.

A typical example of cicatrizing gauzes or bandages is provided by obtaining them with cotton fibre derivatized with peptides obtained from the fragmentation of the fibrinogen (for examples Sigma F 3643).

Another example is a fibre where the conjugated agent is fibronectin (Sigma 2006) or peptides promoting its adhesion (Sigma F 3667).

### 4.c. Anti-sore and repithelializings.

Sores, bedsores and other forms of chronic or chronicizing tissue damage involve several biomechanisms such as inflammation, immune processes, angiogenesis and others.

The therapeutic approach to repair can therefore be different by using substances with a different or multi-factorial effectiveness.

Among the substances which have shown a certain effectiveness on specific biomechanisms involved we can quote:
a. ketanserine (MW 395.40 - Fluka 60712) - R.P. Rooman and H. Janseen, Clin. Exp. Appr: to Derm. and Epiderm. Repair, Eds. A. Barbul and coll., Prog. In Clin. Biol. Res. Vol. 365, 1991, 115, Wiley Liss, NY.
b. hyaluronic acid - N. Scott Adzick and M. T. Longaker, ibid., page 177.
c. Epidermal Growth Factor (EGF - Sigma E 9644) or its peptide fragments (fragment 20-31 Sigma E 9384) - M. Eisinger, R.B. Flick, J. Bizick and Y. Arita, ibid., page 375.
d. Non-specific immunomodulators, such as glycoproteins of microorganic origin - M. Laato, ibid., page. 459.

According to the nature of the tissue damage (wound, bedsore, of other nature), it can be useful to use one or more of the aforesaid active principles.

According to the invention these substances are conjugated singularly with the cotton fibre.

According to the invention the fabric, gauze, etc. can be obtained with one or more fibres or yarns differently derivatized.

### 4.d. Anti-erythema and -dermatopathies with infective and immune matrix.

### Examples of illnesses: atopical and contact dermatitis, urticaria, psoriasis, pemphigus.

The basic idea is always to use cotton fabrics (gauzes) obtained with single or associated fibres derivatized with immunostimulating, cortisone, anti-bacterial agents, etc. according to the causes of the pathology and to its evolution.

### 4.e. Anti-burns due to UV rays or other causes.

The therapeutic principle is the same which has been previously mentioned with the possibility to use fibres derivatized with hyaluronic acid or peptides obtained by degradation of the fibrinogen as repithelializing agents (regenerating the injured or necrotized skin tissue).

### 4.f. Wrinkle-preventing and anti-aging.

Several factors are involved in skin aging processes and related events.

Among these there is certainly a reduced tissue elasticity, a reduced function of the subcutaneous immunocompetent system, a modified morphologic and metabolic pattern of the collagen structure and, according to some researchers, a reduced bioavailability of hyaluronic acid (A. K. Balin and A. M. Kligman, Aging and the Skin, 1989, Raven Press, NY).

The areas which are most involved in these process are generally those subject to traumas and radiations of different nature such as face, neck and legs.

The substances which can be used in the prevention and correction of said events on the basis of known results are: polysaccharides of the type dermatan sulfate (condroitin sulfate B - Sigma C 4259); non-specific immunostimulating agents of bacterial origins, collagenases (Sigma C0130), elastin (Sigma E 1625).

These active principles, conjugated for instance with the cotton fibre, can be applied onto the concerned areas by means of gauzes or other products obtained from cotton, thus obtaining cosmetic products.

### 5. Sanitary cotton wool

### 5.a. Disinfectant.

With fibre derivatized with germicides or anti-bacterial agents, as seen above.

### 5.b. Anesthetic.

With fibre derivatized with local anesthetics according to the example described above.

### 6. Items used in food industry.

Gauzes (bandages) conjugated with disinfectants (for instance benzoic acid), antimycotic agents (for instance nistatin) and/or anti-bacterial agents (for instance sulfadiazine, bacitracin, etc.) can be used to filter cheese or to bandage sausages (salamis) and cheese.

### 7. Anti-mite fabrics.

Fabrics (both, of the woven and of the non-woven type) can be conjugated with anti-mite agents such as to prevent contamination of the said fabrics (which can be used in turn for the manufacture of household articles made from textiles like carpets, curtains, bedclothes, matresses and the like) with mites and more importantly with their metabolites, which harbour a considerable allergenic risk challenging humans which are in continuously exposed to the contact with fabrics, in particular with household articles. It thus can be reasonably estimated that mite-resistant fabrics will display a considerably extended life-time, as far as the considerably lowered allergenic risk is concerned. On the other hand, prior to the present invention, protection against mites would have required impregnation of the fabrics with acaricides, with the inherent risk of quick and uncontrolled release of the active agent. Thus, apart from the danger arising from the possible initial release of toxic doses, achievement of a truly prolonged anti-mite activity in time (and in particular withstanding the cleaning to which most fabrics are subjected during their extended use) was extremely difficult.

The aforesaid problem is solved by the present invention which provides, for the first time, for a polysaccharide polymer conjugated to an active substance having anti-mite activity. Preferred active substances of the invention are hexachlorocyclohexane (in particular gamma-hch (also termed lindane or gammexan), but also the remaining 7 stereoisomers), benzylbenzoate or mesulfene which can be conjugated through suitable linkers or poly linkers of the invention to polysacchraride polymers such as to provide the desired long-term anti-mite effect without release of toxic doses.

### CHEMICAL EXPERIMENTAL PART

### I. Examples of direct conjugation or through the lysyl or hydrazyl linker (COMPARATIVE)

### A. Chemical derivatization of the cotton fibre.

1) 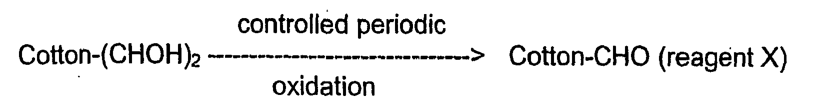
2)

   Cotton-CHO + lysine → Cotton-CH=N-lys + H₂O
3a)

   Cotton-CH=N-CH(COOH)-(CH₂)₃-CH₂-NH₂ + NaBH₄ → Cotton-CH₂-NH-CH(COOH)-(CH₂)₃-CH₂-NH₂ (reagent Y)
3b)

   Cotton-CH=N-CH₂-(CH₂)₃-CH(NH₂)-COOH + NaBH₄ → Cotton-CH₂-NH-CH₂-(CH₂)₃-CH(NH₂)-COOH (reagent Y)

### Quantitative Synthesis.

An aliquot of natural cotton fibre is pretreated by re-suspending it in acetone under mild stirring for 15 minutes, so as to obtain its degreasing (chloroform could also be used to said purpose); then acetone is eliminated, the fibre is washed several times with water and then dried.

Then 20 g of cotton (degreased fibre) are suspended in 1 I of a fresh solution of sodium metaperiodate (Fluka 71859) 0.05 M (10.7 g/l) in distilled water.

The fibre is kept under mild stirring for 5 hours at lab temperature and in absence of light.

The oxidizing solution is then eliminated and the fibre is washed several times with water (the reagent X is obtained, cotton-CHO, the first activated polysaccharide polymer). Since the controlled periodic oxidation of cellulose in the conditions described in the present application leads to the cleavage of the D-glucose ring between two vicinal alcohol groups (which are transformed into two aldehyde groups), said controlled oxidation (because of its specificity for vicinal diols) allows to substantially maintain the molecular chain of cellulose and therefore does not lead to such a fragmentation to destroy the cellulose fibre undergoing activation. Said activated fibre (reagent X) is re-suspended in 500 ml of a solution of lysine HCl (Fluka 62960) in water (1.8 g/500 ml) and the reaction is carried on for 2 hours at room temperature under stirring. In these conditions the Schiff base between the oxidized cotton fibre (reagent X) and, according to the orientation, one of the amino positions of lysine, is obtained as second activated polysaccharide polymer. Therefore, said second activated polysaccharide polymer ("cotton-CH=N-lys") is a mixture of the two Schiff bases which can be obtained, as disclosed above in the following steps 3a and 3b.

Then 1 g of NaBH₄ (Fluka 71320) is added and the reaction is carried on for 1 hour at room temperature under stirring.

The fibre is filtered and washed several times with water (the reagent Y is obtained, i.e. the third activated polysaccharide polymer, which is a mix of the two orientations obtained as in stages 3a and 3b).

### B. Conjugation with the cotton fibre of a bactericide:

A dialdehyde and a phenol derivative are taken as examples of bactericides.

The synthesis scheme is the following:

### Example no. 1 (COMPARATIVE):

10 g of derivatized cotton fibre (reagent Y), re-suspended in 250 ml of carbonate-bicarbonate buffer 0.05 M pH 9.0, are added with 0.3 g (i.e. 1.2 ml of the 25% solution) of glutaraldehyde (Fluka 49624) and are kept under stirring at room temperature for 1 hour.

The fibre is washed several times to eliminate the excess of reagent and then dried.

The following is obtained:

cotton-CH₂-NH-lys-N=CH-(CH₂)₃-CHO

In the present comparative example glutaraldehyde is linked to the polysaccharide polymer through the lysyl linker, with the creation of a secondary amino bond between the activated polysaccharide polymer arid the linker, and of an imino bond between the pharmacologically or biologically active substance and the linker. In turn, a pharmacologically or biologically active substance may be linked to the terminal aldehyde residue.

Yield of conjugated active agent = 82%.

Optionally, according to the needs of bioavailability of the pharmacologically, or biologically active substance, the imino bond of the pharmacological or biologically active substance can then be reduced, for instance with NaBH₄, thus obtaining the corresponding secondary amino bond.

### Example no. 2 (COMPARATIVE):

10 g of derivatized cotton fibre (reagent Y), re-suspended in 250 ml of carbonate-bicarbonate buffer 0.05 M pH 9.0; are added with 0.4 g of 4-hydroxybenzaldehyde (Fluka 54589).

The reaction is carried on under stirring at room temperature for 2 hours, then the fibre is washed several times to eliminate the excess of reagents.

1 g of NaBH₄ (Fluka 71320) is then added, the reaction is carried on at room temperature under stirring for 1 hour; then the fibre is filtered and washed several times with water.

The fibre is then dried.

The following is obtained:

cotton-CH₂-NH-lys-NH-CH₂-Ph-OH

in which Ph is a para-phenylene radical.

Yield of conjugated active agent 74%.

In the present comparative example the pharmacologically or biologically active substance, which is a phenol derivative, is conjugated with the polysaccharide polymer through the lysyl linker, with the creation of two secondary amino bonds.

### C. Conjugation of the cotton fibre with bacteriostats having different structure:

### C.1 Conjugation of the cotton fibre with a sulfamide drug (COMPARATIVE):

10 g of oxidized cotton fibre (reagent X), re-suspended in 250 ml of water, are added with 1 g of sulfadiazine sodium salt (Sigma S 6387) previously dissolved in 10 ml of carbonate-bicarbonate buffer 0.05 M. pH 9.0 and the reaction is carried on at room temperature under stirring for 1 hour.

The obtained conjugate is the Schiff base between the first activated polysaccharide polymer (reagent X) and the amino group of sulfadiazine. In this conjugate the pharmacologically or biologically active substance is linked to the polysaccharide polymer through a double bond, i.e. the imino bond.

Then 1 g of NaBH₄ (Fluka 71320) is added and the whole is kept under stirring for 10 minutes.

The derivatized fibre is washed several times to eliminate the excess of reducing agent and then dried.

The following is obtained:

cotton-CH₂-sulfadiazine

In the present comparative example the pharmacologically or biologically active substance is linked to the polysaccharide polymer through only one bond, i.e: the secondary amino bond.

Yield of conjugated sulfadiazine 87%...

### C.2. Conjugation of the cotton fibre with bacitracin:

10 g of oxidized cotton fibre (reagent X), re-suspended in 250 ml of carbonate-bicarbonate buffer 0.05 M pH 0.9, are added with 0.2 g of bacitracin (Fluka 11792) in powder form.

The reaction is carried on at room temperature under stirring for 2 hours .

1 ml of glycine 2 M (Fluka 50046) are added (150 mg/ml) and the stirring is continued for another hour.

The derivatized fibre is washed several times and then dried.

The following is obtained:

cotton-CH=N-bacitracin

Yield of conjugated bacitracin 94%.

### C.3. Conjugation of the cotton fibre with gramicidin.

The same experimental conditions as in item C.2.) and the same weight ratios are applied.

The following is obtained:

cotton-CH=N-gramicidin

Yield of conjugated gramicidin 88%.

### D. Conjugation of local anesthetic with the cotton fibre:

In the present case procaine is chosen as an example.

The conjugation reaction is the following:
10 g of oxidized cotton fibre (reagent X) are re-suspended in 250 ml of carbonate-bicarbonate buffer 0.05 M pH 9.0, and are added with 0.2 g of procaine HCl (Fluka 81666), dissolved in 5 ml of distilled water, under stirring.

The reaction is carried on for 2 hours at room temperature, then the aldehyde sites which are still free are blocked by adding 1 ml of glycine 2 M; the reaction is carried on for 1 hour at room temperature.

The fibre is then washed several times to eliminate the excess of reagents not linked to the fibre, and then dried.

The following is obtained:

cotton-CH=N-procaine

In the present comparative example the pharmacologically or biologically active substance is linked to the polysaccharide polymer through a double bond, in particular the imino bond of the Schiff base formed between the aldehyde function of the activated polysaccharide polymer and the amino group of the pharmaceutically or biologically active substance.

### Yield of conjugated procaine 85%.

### E. Conjugation scheme of the cotton fibre with coagulating factors with macromolecular structure:

### E.1. Conjugation with F VIII (anti-hemophilic plasmatic factor):

### (a) Derivatization of the protein:

### Formation of the hydrazide derivative.

10 g of F VIII are dissolved in 200 ml of water and are added with a solution of dihydrazide of adipic acid 0.5 M (Fluka 02191) (87 g/l - 20 ml) with pH 5 and the volume is brought to 250 ml with water.

An aqueous solution of 1-ethyl-3-(3-dimethylaminopropyl)carbodiimide (Sigma E 1769), ("EDAC") (1 g in 5 ml of acetate buffer with pH 5.6) is added and the reaction is carried on for 6 hours at room temperature under stirring, the pH value being kept below 5 by adding hydrochloric acid.

The whole is then dialised against carbonate/bicarbonate buffer 10 mM pH 9.

### (b) Conjugation reaction:

The dialyzed product is added to 10 g of oxidized cotton fibre (reagent X) re-suspended in 250 ml of carbonate-bicarbonate 0.05 M pH 9.0.

The reaction is carried on for 2 hours at room temperature under stirring.

Then 1 g of NaBH₄ is added and the reaction is carried on for 1 hour at room temperature under stirring.

The fibre is filtered and then washed several times to eliminate unreacted compounds.

The fibre is then dried.

The following is obtained:

cotton-CH₂-NH-NH-C(O)-FVIII

Yield of conjugated F VIII 72%.

In the present comparative example the conjugation between the polysaccharide polymer and the pharmacologically or biologically active substance has been established through the linker hydrazine. The hydrazyl linker has been introduced by means of derivatization of the pharmacologically or biologically active substance. The conjugation thus obtained comprises (beyond a secondary amino bond and a peptide bond) a single bond between two nitrogen atoms.

### E.2. Conjugation of the fibrinogen.

10 g of activated cotton fibre (reagent Y) are suspended in 250 ml of carbonate-bicarbonate buffer 0.05 M pH 9.0.

0.1 g of fibrinogen (Sigma F 3879) in 1 ml of the same buffer and immediately afterwards 0.5 g of 1-ethyl-3-(3-dimethylaminopropyl)carbodiimide (Sigma E 1769), ("EDAC") in powder are added. The reaction is carried on under stirring at 4°C for twelve hours, constantly controlling the pH value at 5.5 with HCl 0,1 N during the first hour.

The reaction is blocked by adding 1 ml of glycine 2 M, carrying the reaction on for further 2 hours at 4°C.

The reaction is then washed several times with water to eliminate unreacted reagents.

The whole is dried and the following mixture is obtained:

cotton-CH₂-NHCH₂-(CH₂)₃-CH(CO-NH-fibr)-NH-CO-fibr

and

cotton-CH₂-NH-(CO-NH-fibr)CH-(CH₂)₃-CH₂-NH-CO-fibr

Yield of conjugated fibrinogen 95%. An example is obtained in which the lysyl linker (statistically introduced according to the two possible orientations), because of the two peptide bonds with two equivalents of pharmacologically or biologically active substance, is a trivalent linker.

### F. Conjugation scheme of an immunostimulating glycoprotein (i.e. association of a peptido glycane with a glycoprotein) obtained from Corynebacterium granulosum, parvum or catarrhalis.

10 g of oxidized cotton fibre (reagent X) are suspended in 250 ml of carbonate-bicarbonate buffer 0.05 M pH 9.0.

0.2 g of suspension of the immunostimulating fraction (obtained in lab according to the method described by B. Bizzini as indicated above) in the same buffer are then added.

The reaction is carried on for 6 hours under stirring at room temperature.

1 g of NaBH₄ is then added and the reaction is carried on for 1 hour under stirring at room temperature, then the derivatized cotton is carefully washed with water and dried.

A derivatized cotton fibre is obtained, in which the conjugation between the polysaccharide polymer and the immunostimulating glycoprotein is established through a secondary amino bond between said polysaccharide polymer and the terminal amino group of the glycoprotein (obviously, if the glycoprotein contains aminated amino acids such as e.g. lysine, also the "lateral" amino groups statistically form secondary amino bonds).

### II. Examples of conjugation through conjugation with cotton-S-S-cysteine (i.e. with two linkers:

### -S- and cysteine, linked one to the other by means of disulfuric bond) and through conjugation with cotton-S- (i.e. by means of a sulfur linker).

### A. Chemical derivatization of the cotton fibre (introduction of SH groups):

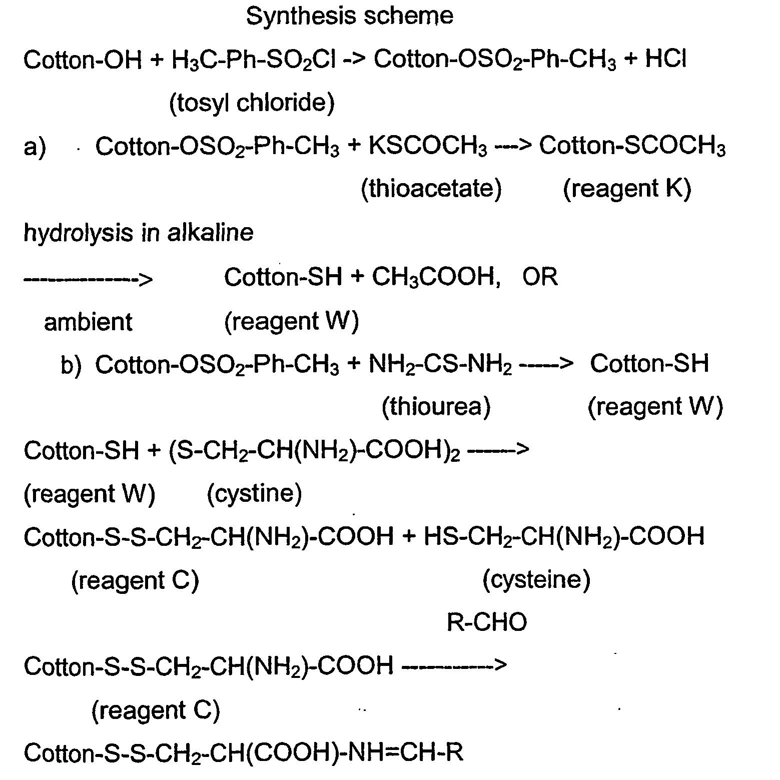

### Quantitative synthesis.

### Example no. 1: Derivatization with tosyl chloride.

10 g of degreased cotton fibre, suspended in a mixture of 200 ml of phosphate buffer 0.1 M pH 8.5 and 100 ml of dioxane, are treated with 7 g of tosyl chloride (Sigma T3, 595-5) at room temperature under stirring for 4 hours.

The derivatized cotton is washed several times with water before being re-suspended in 200 ml of acetate buffer 0.1 M pH 5.5, containing potassium thioacetate 0.5 M (11 g).

The trans-esterification is carried on for 24 hours at room temperature, then the cotton is washed several times with water.

The deacetylation of the cotton thioaceto-derivative has been obtained by adjusting the pH value at 12 of the cotton suspension in 200 ml of water.

The thio-derivative is washed several times with water and then dried.

The following is obtained:

Cotton-SH (reagent W)

### Example no. 2: Derivatization of cotton-SH with cysteine

10 g of cotton-SH suspended in 200 ml of carbonate-bicarbonate buffer 0.5 M pH 9.0 are added with 1.5 g of L-cystine 2HCl (Sigma C 2526) dissolved in 20 ml of water.

The reaction is carried on under stirring for 4 hours at room temperature.

The cotton is then washed several times with water and dried.

The following is obtained:

cotton-S-S-CH₂-NH(NH₂)-COOH (reagent C)

Yield of the reaction: 86%

In the present activated cotton derivative the polysaccharide polymer has been conjugated with the linker -S- (or sulfur linker), formally derived from H₂S, which has been in turn conjugated with the cysteinyl linker through a disulfur bond. Since there is only one possibility for the formation of a disulfur bond, differently from the lysyl linker as described above, the cysteinyl linker reacts with the activated polysaccharide polymer according to one only orientation.

### B. Conjugation of fibrinogen with the cotton fibre.

10 g of cotton-S-S-cysteine (reagent C) suspended in 200 ml of acetate buffer 0.1 M pH 5.5 are added with 0.1 g of fibrinogen (Sigma F3879) in 10 ml of the same buffer and immediately afterwards with 0.5 g of 1-ethyl-3-(3-dimethylaminopropyl)-carbodiimide (Sigma E 1769), ("EDAC") in powder.

The reaction is carried on under stirring at room temperature for 4 hours, constantly adjusting the pH value at 5.5 with HCl 0.1 N during the first hour.

After the reaction the cotton is washed several times with water and then dried.

The following is obtained:

cotton-S-S-GH₂-CH(NH-CO-fibr)-CONH-fibr .

Yield of bound fibrinogen 68%.

In the present comparative example the pharmacologically or biologically active substance has been conjugated with the polysaccharide polymers through two linkers, the first of which consists of -S- (formally obtained from H₂S) and the second is the cysteinyl linker (formally obtained from cysteine). In the present case, because of the two peptide bonds between the cysteinyl linker and the pharmacologically or biologically active substance, the cysteinyl linker constitutes a trivalent linker.

### C. Conjugation of coag. Factor VIII with cotton fibre

The conjugation scheme is the same used above for fibrinogen.

The yield of conjugated Factor VIII is of 64%.

### D. Conjugation of the cotton fibre with heparin with low molecular weight (MW)

### 1. Periodic oxidation of heparin:

0.25 g of sodium heparin (Sigma H 1636) are dissolved in 10 ml of acetate buffer 0.01 M containing 0.01. M of NalO₄.

The solution is stirred in absence of light at room temperature for 20 minutes.

The reaction is stopped by adding 100 µl of glycerol kept in contact for 15 minutes under constant stirring.

Oxidized heparin is then dialyzed against carbonate-bicarbonate Buffer 0.1 M pH 9.0.

Similarly to the case of the polysaccharide polymer described under item l above, also here the controlled oxidation with metaperiodate (which is specific for vicinal diols) results in two aldehyde groups on the pharmacologically or biologically active substratum, with cleavage of the ring of d-glucuronic acid contained in heparin in the positions occupied by vicinal secondary alcohol groups.

### 2. Conjugation of oxidized heparin with cotton-S-S-cysteine:

5 g of cotton-S-S-cysteine (reagent C) suspended in 100 ml of carbonate-bicarbonate buffer 0.1 M pH 9.0 are reacted with 0.25 g of oxidized heparin under stirring at room temperature for 3 hours.

After the reaction is completed, the cotton is washed several times with water before being dried.

The following is obtained:

cotton-S-S-CH₂-CH(COOH)-N=CH-heparin

In the present comparative example the polysaccharide polymer, which has been activated by conjugation with the linker -S- and the cysteinyl linker to obtain the reagent C, is conjugated with the pharmacologically or biologically active substance through the formation of the Schiff base between an aldehyde group created by derivatization of the pharmacologically or biologically active group and the amino group of the cysteinyl tinker.

As an alternative, the conjugation between the activated polysaccharide polymer (reagent C) can also take place without modifying heparin as pharmacologically or biologically active substance, through the residue of glucuronic acid of heparin which can establish (namely through the carbodiimide method, e.g using "EDAC") a peptide bond with the amino group of the reagent C.

Yield of heparin with low MW conjugated through the formation of the Schiff base with cotton-S-S-cysteine: 74%.

### E. Conjugation of the cotton-S-S-cysteine fibre with naproxen (anti-inflammatory).

10 g of cotton-S-S-cysteine (reagent C) suspended in 200 ml of acetate buffer 0.1 M pH 5.5 are added with 10 ml of an aqueous solution containing 0.25 g of naproxen (Sigma N 5160), and immediately afterwards with 0.5 g of 1-ethyl-3-(3-dimethylaminopropyl)carbodiimide (Sigma E 1769), ("EDAC").

The mixture is stirred at room temperature for 4 hours, adjusting the pH value at 5.5 with HCl 0.1 N during the first reaction hour.

The fibre is washed several times to eliminate the excess of reagents and then dried.

The following is obtained:

cotton-S-S-CH₂-CH(COOH)-NH-CO-naproxen

Therefore, in the present comparative example the conjugation between the activated polysaccharide polymer and the pharmacologically or biologically active substance carrying a carboxyl group occurs through the formation of a peptide bond between the reagent C and the pharmacologically or biologically active substance.

Yield of conjugated anti-inflammatory 67%.

### F. Conjugation of cotton-S-S-cysteine with lipase.

10 g of cotton-S-S-cysteine (reagent C) in 200 ml of acetate buffer 0.1 M. pH 5.5 are added with 0.1 g of lipase (Sigma L 1754) in 10 ml of the same buffer, and immediately afterwards with 0 0.5 g of 1-ethyl-3-(3-dimethylaminopropyl)carbodiimide (Sigma E 1769), ("EDAC") in solid form,

The reaction is carried on at 4°C under stirring for 4 hours, adjusting the pH value at 5.5 during the first reaction hour, if necessary.

After the reaction the derivatized cotton is washed several times, then dried and kept at 4°C.

The following is obtained:

cotton-S-S-CH₂-CH(NH-CO-lipase)-CO-NH-lipase

In the present comparative example as well, the cysteinyl linker is a trivalent linker.

Yield of conjugated lipase on the basis of lipase activity: 65%

### G. Conjugation of IgG (immunoglobulins) with the cotton-S-S-cysteine fibre.

### 1. Reduction of IgG:

100 mg of IgG in 1 ml of PBS (phosphate/saline buffer) pH 7.4 containing 1 mM of EDTA are reduced by treatment with DTT (Sigma D 9680) to a final concentration of 5 mM at room temperature for 20 minutes, breaking the disulfur bridges which are present on the IgG and regenerating the corresponding thiol groups of the cysteines contained in the peptide chain of the IgG, thus obtaining IgG-SH".

The solution is then filtered on a column of Sephadex G 25 and the fraction of reduced IgG ("IgG-SH") is collected.

### 2. Reaction of reduced IgG with cotton-S-S-cysteine

10 g of cotton-S-S-cysteine (reagent C) suspended in 200 ml of PBS-EDTA are treated with 100 mg of reduced IgG (IgG-SH) in 12 hours under stirring in nitrogen atmosphere.

The cotton is then washed several times and dried.

The following is obtained:

cotton-S-S-IgG

Yield of conjugated IgG 67%.

Therefore, in the present comparative example the cysteinyl linker of the reagent C reacts as a leaving group and is removed and replaced by the thiol groups which are present on the pharmacologically or biologically active protein, thus forming a disulfuric bond between the reagent W (as activated polysaccharide polymer) and the pharmacologically or biologically active protein. A conjugate is therefore obtained, in which the polysaccharide polymer is conjugated with the pharmacologically or biologically active substance by means of the linker-S-.

### H. Conjugation of hyaluronic acid with cotton-S-S-cysteine.

### Method no. 1:

5g of cotton-S-S-cysteine (reagent C) suspended in 100 ml of acetate butter 0.1 M pH 5.5 are added with 10 ml of a solution containing 0.1 g of sodium hyaluronic acid (Sigma H 1876) in water, and immediately afterwards with 0.5 g of 1-ethyl-3-(3-dimethylaminopropyl)carbodiimide (Sigma E 1769), ("EDAC").

The reaction is carried on under stirring at 4°C for 4 hours, adjusting the pH value at 5.5 during the first reaction hour by adding HCI 0.1. N.

After the reaction the cotton is washed several times and then dried.

The following is obtained:

cotton-S-S-CH₂-CH(COOH)-NH-CO-hyaluronic

Therefore, in the present comparative example a peptide bond is established between the amino group of the reagent C and the carboxyl group which is present on the hyaluronic acid.

Yield of conjugated hyaluronic acid 57%.

### Method no. 2: Oxidation of hyaluronic acid:

100 mg of sodium hyaluronic acid (Sigma H 1876) are dissolved at 4°C arid in absence of light in 10 ml of buffer acetate 0.1 M pH 5.5 containing 1 mM of NalO₄.

Then the whole is stirred for 15 minutes.

The reaction is then blocked by adding 100 µl of glycerol which is then reacted under stirring for further 15 minutes.

### Conjugation:

5 g of cotton-S-S-cysteine (reagent C) suspended in 100 ml of carbonate-bicarbonate buffer 0.1 M pH 9.0 are added with the solution of oxidized hyaluronic acid and the reaction is carried on under stirring at 4°C for 4 hours.

The derivatized cotton is then washed several times with water and dried.

The following is obtained:

cotton-S-S-CH₂-CH(COOH)-N=CH-hyaluronic

As in the case of heparin described above, also hyaluronic acid can be oxidized with sodium metaperiodate with the creation of two aldehyde groups which can be conjugated with the reagent C through the formation of the corresponding Schiff base.

Yield of conjugated polysaccharide: 45%.

### I. Conjugation of collagenase with cotton-S-S-cysteine.

5 g of cotton-S-S-cysteine suspended in 100 ml of acetate buffer 0.1 M pH 5.5 are added with 1 ml of a solution in PBS 0.01 M containing 0.1 g of raw collagenase type 1A (Sigma C 9891), and immediately afterwards with 0.5 g of 1-ethyl-3-(3-dimethylaminopropyl)carbodiimide (Sigma E 1769), ("EDAC") in powder.

The reaction is carried on under stirring at 4°C for 4 hours, adjusting the pH value at 5.5 during the first 20 minutes with HCl 0.1 N.

After the reaction the cotton is washed several times with very cold water.

The cotton is then dried.

The following is obtained:

cotton-S-S-CH₂-CH(NH-CO-coll)-CO-NH-coll

Yield on the basis of the hydrolytic activity on FALGPA substratum (Sigma F 5136): 53%.

### J. Conjugation of elastin with cotton-S-S-cysteine.

10 g of cotton-S-S-cysteine (reagent C) fibre suspended in 200 ml of acetate buffer 0.1 M pH 5.5 are added with 0.1 g of elastin (Sigma E 1625) in 10 ml of the same buffer, and then with 0.5 g of 1-ethyl-3-(3-dimethylaminopropyl)carbodiimide (Sigma E 1769), ("EDAC") in powder.

The reaction is carried on at 4°C under stirring for 4 hours, adjusting the pH value at 5.5 for the first 20 minutes with HCl 0.1 N.

After the reaction the derivatized cotton is washed several times to eliminate the unreacted reagents.

The cotton is then dried.

The following is obtained:

cotton-S-S-CH₂-CH(NH-CO-elast)-CO-NH-elast

Yield: undetermined.

### III. Examples of conjugation between polysaccharide polymer and pharmacologically or biologically active substance through the linker obtained from 4-diazobenzoic acid.

### A. Conjugation of cortisol ("hydrocortisone", 11β,17α,21-trihydroxypregn-4-ene-3,20-dione, Sigma H4001) with the cotton fibre (COMPARATIVE).

### Derivatization of hydrocortisone with the diazo-derivative of p-aminobenzoic acid.

### a) Preparation of the diazo-derivative of p-aminobenzoic acid (PABA).

548 mg of PABA are dissolved in 40 ml of HCl 1 N and the solution is cooled on ice.

248 mg of NaNO₂ dissolved in 20 ml of iced water are added dropwise under stirring to the solution of PABA, then the stirring is carried on for another hour in an ice bath.

### b) Derivatization of hydrocortisone.

1,300 mg of hydrocortisone are dissolved in a mixture of 20 ml of acetic acid and 90 ml of dimethylformamide. This solution is added dropwise with the solution of the diazo-derivative of PABA under stirring, operating in an ice bath in absence of light. The stirring is carried on at 4°C for 12 hours, then the diazo-derivative of hydrocortisone is precipitated by adding a great volume of water. The precipitate is collected by centrifugation, re-dissolved in ethyl acetate and washed twice with water. The organic phase is collected on Na₂SO₄, filtered and evaporated until dryness.

### Conjugation of the diazo-derivative of hydrocortisone with the cotton fibre.

20 g of cotton-S-S-cysteine (reagent C) suspended in dioxane are added with 150 mg of 1-cyclohexyl-3,2-morpholino-ethylcarbodiimide methyl-p-toluensulfonate (Sigma C1011), then with 100 mg of the azo-derivative and the reaction is carried on under stirring at 4°C for 12 hours. The cotton is then washed, first with dioxane, then several times with water before being dried.

The following is obtained:

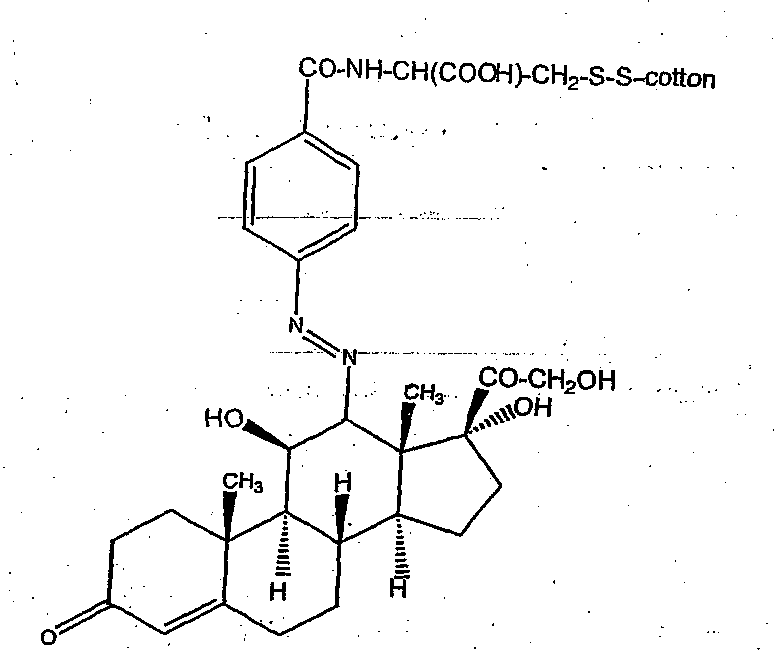

The conjugate thus obtained is an example of the use of 4-azobenzoic add as linker in the present invention. Such linker is introduced by derivatization of the pharmacologically or biologically active substance. A chemical conjugation is thus obtained between the polysaccharide polymer and the pharmacologically or biologically active substance comprising a double bond between two nitrogen atoms.

Yield : 064%.

### B. Conjugation of benzyl benzoate (MW 212,3 -Sigma B 9550 -USP-Merck Index 11,1141, CAS-number 120-51-4): C₆H₅CO₂CH₂C₆H₅

Suitably activated benzyl benzoate may be conjugated to cotton fibre through linkers (like e.g. lysine or cysteine) or through polylinkers (like e.g. poly L-lysine MW 4000-15000 Sigma P6516, poly D-lysine MW 1000-4000 Sigma P 0296 or polyglutamic acid Sigma P 4636 MW 3000-15000) or combinations thereof.

### 1. Activation of benzyl benzoate

### 1.1. Preparation of the diazo derivative of p-aminobenzoic acid (PABA Sigma A 9878).

274 mg of PABA are dissolved in 20 ml of 1 N HCl, and the solution thus obtained is cooled on ice. 124 mg NaNO₂ (Sigma S 2252) are dissolved in 10 ml ice water and the solution is added dropwise, under stirring, to the PABA solution, where after the stirring is continued for an additional hour, always under ice cooling.

### 1.2. Derivatization of benzyl benzoate

424 mg of benzyl benzoate are dissolved in a mixture of 20 ml acetic acid and 80 ml ethanol. To this solution, the aforementioned solution of the diazoderivative of PABA is added dropwise, under stirring and under exclusion of light. The stirring is continued for 18 hours at 4° C. Thereafter, the azo derivative of benzyl benzoate is precipitated through addition of a huge amount of water. The precipitate is collected through centrifugation, dissolved in a small volume of ethanol and again precipitated through addition of water. The obtained product is again centrifuged, dissolved in ethanol and than dried through evaporation.

One obtains:

Ph-COOCH₂-Ph-N=N-Ph-GOOH (isomer I)

and

HOOC-Ph-N=N-Ph-COOCH₂-Ph (isomer II)

wherein -Ph- designates a para-phenylene radical. Such isomeric mixture is designated as "PABA-benzylbenzoate" in the following.

### 2. Conjugation of the azo derivatives with cotton fibre:

### 2.1. Conjugation through the lysylic linker (COMPARATIVE)

For the conjugation, lysylated cotton, either not reduced with NaBH₄ ("non-reduced reagent Y" i.e. wherein a Schiff base has been established through reaction of the aldehyde group on the activated polymer and an amino group of lysine) or reduced with NaBH₄ (i.e. reagent Y as above) may be used, depending on whether the active substance is supposed to become released or not

To 20 g of derivatized cotton (reduced or non-reduced reagent Y), suspended in dioxane, are added 400 mg 1-cyclo hexyl-3,2-morpholinoethyl-carbodiimmide methyl-p-toluene sulfonate (Sigma C1011), followed by 280 mg of the previously obtained azo derivative and the reaction is left stirring at 4° C for 18 hours. The cotton is then washed, first with dioxane, then repeatedly with water, and dried.

One obtains:
- (2.1.1.) with non-reduced reagent Y:

   cotton-CH=N-lys-NH-CO-PABA-benzylbenzoate,

   which is a mixture of isomers since both of the isomers I and II as above get linked to non-reduced reagent Y (harbouring in turn two different orientations of the lysyl linker).
   Yield: 72%
- (2.1.2.) with reduced reagent Y:
   cotton-CH₂-NH-lys-NH-CO-PABA-benzyl benzoate, which is likewise a mixture of isomers since both of the isomers I and II as above get linked to reduced reagent Y (harbouring in turn two different orientations of the lysyl linker).
   Yield: 67%.

### 2.2. Conjugation through polylinker (INVENTION):

### Preparation of a conjugate between cotton-poly-D-lysine and the azo derivatives of benzyl benzoate:

Activated cotton fibre, of the type of reagent Y and non-reduced reagent Y were used, except for the fact that instead of lysine, poly D-lysine (Mw 1000-4000, Sigma P 0296) was conjugated to the fibre. The further synthetic protocol corresponded, *mutatis mutandis,* to the one set out above under issue 2.1. In particular, the following was obtained:
- (2.2.1.) with non-reduced activated cotton fibre:

   cotton-CH=N-polylys-(NH-CO-PABA benzylbenzoate)_{n,}

   which is an isomeric mixture on the grounds set out above.
   yield 97%
- (2.2.2.) with reduced activated cotton fibre :

   cotton-CH₂-NH-polylys- (NH-CO-PABA-benzylbenzoate)_{n,}

   which is likewise an isomeric mixture on the grounds set out above.
   yield 100%

### 3. Table displaying obtainable yields:

### Conjugation ratio of benzyl benzoate to cotton fibre using various methods:

| Conjugate | Ratio benzyl benzoate /cellulose mg/gr in % |
|---|---|
| III.B.2.1.1. (comparative) | 72,0 |
| III.B.2.1.2. (comparative) | 67,0 |
| III.B.2.2.1. (invention) | 97,0 |
| III.B.2.2.2. (invention) | 100 |

The conjugates as above are examples for polysaccharide polymers conjugated to acaricide molecules (i.e. anti-mite agents) through preferred linkers of the invention, in particular a poly lysyl linker.

In particular, conjugate IIIB2.2.2. is constituted by a polysaccharide polymer conjugated to PABA-derivatized benzylbenzoate through a poly lysyl linker having a molecular weight of less than 16000, preferably 1000-4000, in Which a secondary amino bond is established between the poly lysyl linker and the polysaccharide polymer, and peptide bonds are established between the poly lysyl linker and the PABA-derivatized benzylbenzoate.

### PHARMACOLOGICAL EXPERIMENTAL PART

### 1. Test for validating the effectiveness of tampons in cotton fibre conjugated with F VIII, fibrinogen and fibrin on the speed and consistency of the in-vitro formation of a blood clot.

The influence of the presence of a cotton tampon derivatized with specific protein on the speed and consistency of a blood clot is determined according to the method developed by Lee/White (Amer J. Med., 1913, 145, 495) and modified. Precisely weighed amounts (about 1 g) of normal cotton wool fibre or of derivatized cotton fibre are introduced in 15 ml test-tubes for serology following the procedures disclosed in the chemical experimental part, together with the various proteins involved in the coagulation reaction, and 5 ml of physiological solution are added to each tube.

The whole is kept in a thermostatic water-bath at 37°C.

Then 5 ml of whole blood obtained by venous puncture from the marginal vein of rabbit ears are poured into each tube, the tubes are lightly stirred and a group of them is then kept under thermo-regulation at 37°c for 5 minutes, whereas the other group for 10 minutes.

After such times cotton wads are taken from the single tubes, dried by light contact with filter paper and weighed.

The following table shows the percentages referring to the increase of the weight of cotton wads conjugated with the various proteins with respect to control percentages (non-conjugated wads).

| Type of conjugate | % difference in the weights with respect to unbound cotton | |
|---|---|---|
| | 5 minutes | 10 minutes |
| Unbound cotton (control) | ― | ― |
| Cotton bound with F VIII (examples IE1 and IIC) comparative | +30.5 | +55.6 |
| Cotton bound with F VIII through poly lysine (MW 1000-4000) (secondary amino ond/peptide bonds) invention | +74,0 | +85,4 |
| Cotton bound with prothrombin (linker: lysine) (secondary amino bond/peptide bonds) comparative | +25.4 | +40.2 |
| Cotton bound with prothrombin (linker: poly-lysine, MW 1000-4000) (secondary amino bond/peptide bonds) invention | +68,0 | +82,5 . |
| Cotton bound with fibrinogen (examples IE2 and IIB above) comparative | +28.6 | +44.6 |
| Cotton bound with fibrinogen (linker: poly-lysine, MW 1000-4000) (secondary amino bond/peptide bonds) invention | +87,2 | +98,0 |
| Cotton bound with FDP (linker: lysine) (secondary amino bond/peptide bonds) comparative | +34.5 | + 53.0 |
| Cotton bound with FDP (linker: poly lysine, MW 1000-4000) (secondary amino bond/peptide bonds) invention | +72,6 | +87,9 |

The results show that the presence of cotton fibre suitably derivatized can accelerate the coagulating process. Moreover, experiments carried out with the same active substance, though conjugated in different ways with the cotton fibre (as in the examples above) have shown that the nature of the conjugation between the aforesaid coagulating proteins and the cotton does not affect the coagulating power of the cotton thus obtained, as far as comparatively short bi- and trifunctional linkers are concerned. However, considerably increased coagulation power may be obtained through the use of poly-linkers of the invention, in particular through the use of the poly lysine linker having a MW of less than 16000, preferably 1000-4000.

This indicates that the derivatized cotton according to the present invention can oppose functional hemorrhages or hemorrhages due to wounds.

### 2. Evaluation of the influence of cotton derivatized with germicides, with anti-bacterial or anti-fungi agents in the development and amount of microorganic flora.

The influence of suitably derivatized cotton fibre on the development of microorganic flora has been determined "in vitro" on plate growing cultures of various micro-organisms: Candida albicans, Escherichia coli, Staphylococcus aureus isolated and characterized by contaminated organic material.

To this purpose growth selective mediums are prepared (sabouraud agar for fungi and nutrient agar for bacteria) with which Petri dishes are prepared.

On the medium surface the microorganism culture is applied by deposition of one ml of a suspension of said microorganism containing 10⁹ microorganisms, lightly agitating so as to cover the whole plate surface.

A cotton gauze obtained from fibre derivatized with the various substances taken into consideration is then applied onto the surface; the whole is then placed in a thermostat at 37°C.

After 24 hours the gauze is removed and the developed colonies are compared with plates in normal growth conditions.

The reduction in percentage of the growth in the plates characterized by conjugated gauze is evaluated with respect to normal growth conditions.

| Product | % reduction in the development of colonies | | |
|---|---|---|---|
| | C. albicans | E. coli | S. aureus |
| Fibre with derivative of 4-hydroxybenzaldehyde Linker: lysine (example I.B.2 above) comparative | 75 | 85 | 85 |
| Fibre derivatized with 4-hydroxybenzaldehyde (linker: poly lysine MW 1000-4000) (secondary amino bonds both, between tinker and polymer and between linker and pharmacologically, active substance molecules) invention | 94 | 100 | 100 |
| Fibre with sulfadimethoxine (Schiff base between amino group of sulfadimethoxine and reagent X) comparative | 60 | 70 | 80 |
| Fibre with sulfadimethoxine (linker: poly lysine MW 1000-4000) Schiff base both, between linker and polymer and between linker and pharmacologically active substance molecules invention | 84 | 100 | 94 |
| Fibre with bacitracin (example I.C.2. above) comparative | 65 | 75 | 95 |
| Fibre with bacitracin (linker: poly lysine MW 1000-4000) Schiff base both, between linker and polymer and between linker and pharmacologically active substance molecules invention | 100 | 100 | 100 |
| Fibre with gramicidin (example I.C.3. above) comparative | 65 | 80 | 90 |
| Fibre with gramicidin (linker: poly lisine MW 1000-4000) Schiff base both, between linker and polymer and between linker and pharmacologically active substance molecules invention | 100 | 100 | 100 |

The results show that the application of cotton derivatized with suitable drugs or germicides on the surface can antagonize the growth on said surface (for instance skin or mucosa) of pathogenic or unwanted microorganic forms.

### 3. Influence of fibre derivatized with anti-inflammatories or with immunostimulating agents on the phlogogenic process.

The experiments have been carried out on rats through the test of induction of granuloma from "cotton pellets".

According to this test cotton pellets having a pre-estabtished weight (1 g) and shape are introduced into the subcutaneous region of the subaxillary area of the animals using a cannula which can pierce the skin and the sub-skin (the scar is closed again with a suture stitch).

Around the cotton pellet a "granuloma" collagen deposit is formed, whose entity is proportional to the phlogogenic process.

After fifteen days from the operation the granuloma is taken from the animal under anesthesia and it is weighed to determine its entity.

In the present case the cotton used as a base for the implantation of the granuloma consists of:
- normal cotton fibre (control),
- cotton fibre derivatized with naproxen (example, II, E. above),
- cotton fibre derivatized with immunostimulating .glycoprotein from C. granulosum (example I.F. above).

The following table shows the difference in percentage of the weight of the granulomas which are formed within the cotton pellets derivatized with anti-inflammatories or immunostimulating agents (from Corynebacterium granulosum) with respect to those within pellets consisting of simple cotton (controls).

| Product | % reduction in the weight of the granuloma |
|---|---|
| Normal cotton fibre (control) | ----- |
| Cotton fibre conjugated with naproxen (example II.E. above) comparative | 30.0 |
| Cotton fibre conjugated with naproxen (linker: cysteyl poly lysine MW 1000-4000): cotton-S-S-cys-polylys-naproxen invention | 64,0 |
| Cotton fibre conjugated with hydrocortisone (example III.A. above: cotton-S-S-cys-PABA-hydrocortisone) comparative | 55.0 |
| Cotton fibre conjugated with hydrocortisone : cotton-S-S-cys-po)ytys-(PABA-hydrocortisone)ₙ invention | 90,0 |
| Cotton fibre conjugated with immunostimulating agent (example I.F. above) comparative | 58.0 |
| Cotton fibre conjugated with immunostimulating agent (immunostimulating glycoprotein) linker: poly lysine Mw 1000-4000 invention | 86,0 |

The results show that the derivatization of the cotton fibre with substances having an anti-inflammatory or immunostimulating activity can antagonize the progression of the experimental chronic phlogogenesis. Whereas, in the above table, in the case of the hydrocortisone and of the immunostimulating glycoprotein, the obtainable improvements may be ascribed to the leverage effect and possibly to the spacer effect provided by the respective poly tinkers, the first couple of experiments comparing two different conjugations of naproxen shows that, surprisingly, a poly lysine tall, in particular a cysteyl poly lysine tall may increase efficacy of naproxene as such, once the disulfuric bond has been cleaved. After the application onto the skin this results in an anti-inflammatory and anti-trauma effect.

### 4. Influence of the application of derivatized cotton fibres on the entity of experimental sclerosis in rabbits' femoral veins.

After a suitable depilation a rabbit's femoral vein is closed for a length of about 5 cm by means of suture stitches downstream and upstream.

Within the separated area 0.05 ml of carbon tetrachloride in 10% solution (Fluka 87031) is added as sclerotizing agent.

The area is suitably bandaged with normal cotton gauzes (control) or derivatized gauzes so that they perfectly adhere to the treated area.

After ten days the vein fragments undergoing stasis are taken and controlled with a microscope to check the parietal state and the consistency of the sclerotizing deposits.

It is thus possible to observe how the application in the area receiving layers of derivatized cotton gauzes with:
- heparin with low molecular weight (comparative example II.D.2.),
can reduce the tissue sclerotic state to a consistent extent. In particular, no difference is observed between the activity of heparin oxidized and conjugated through the formation of a Schiff base and heparin conjugated through peptide bonds with the intervention of the groups of glucuronic acid which are present on heparin. However, efficacy can be substantially increased if more equivalents of low molecular weight heparin are conjugated through peptide bonds to cotton previously derivatized with poly lysine. The same strategy may be pursued with oxidized heparin which is able to form Schiff bases with the amino groups of the poly lysine chain.

It can thus be inferred that the application of said gauzes has a pharmacological effect in the regulation of vascular functions and in the formation and consistency of venous thrombi.

This shows the importance of said application in the prevention and therapy of varices and of phlebitic pathogenesis.

### 5. Influence of the application of derivatized cotton fibres on time of experimental bleeding in rabbits' ears.

A rabbit's ear is suitably depilated and washed with physiological solution.

Then some cuts are made on the surface in horizontal direction using a normal razor-blade, trying to use as much uniformity as possible for the various animals. Immediately afterwards cotton gauzes, both normal (controls) or obtained from fibre derivatized with pro-coagulating factors or peptides (F VIII or fibrinogen degradation products, "FDP"), are applied onto the wounds.

The blood spot on the gauze is controlled.

Its reduction indicates a cicatrizing property.

The following table shows the reductions in percentage of the spots which can be found in derivatized gauzes with respect to controls. In this case as well no difference due to the different nature of the chemical conjugations in the various preparations of cotton derivatized with factor VIII or fibrinogen can be observed. Notwithstanding the foregoing, an additional benefit is again obtained through the use of the poly lysyl linker.

| Product | % reduction of the blood spot |
|---|---|
| Controls | ----- |
| Gauzes derivatized with con F VIII (linker: hydrazine, cysteine see examples I E1 and IIC respectively, above) comparative | 60 |
| Gauzes derivatized with FVIII (linker: poly lysine, secondary amino bond/peptide bonds) invention | 90 |
| Gauzes derivatized with FDP (linker: lysine, secondary amino bond/peptide bonds) comparative | 55 |
| Gauzes derivatized with FDP (linker: poly lysine, MW 1000-4000, secondary amino bond/peptide bonds) invention | 92 |

### 6. Influence of the application of derivatized cotton fibres on UV-induced edema in rats' skin

Adult rats are depilated on their backs and are then exposed to UV radiations until a consistent skin edema forms.

The exposition is carried out only on pre-established circular areas using suitable pierced masks.

Then normal cotton gauzes (controls) or gauzes obtained from fibre derivatized with a medicament are applied with a good adherence onto the inflamed areas.

Simultaneously another group of animals is tested to check the effectiveness of the application of the same medicaments in form of 5% gel.

The regression of the erythema defined as the reduction of the entity and surface of the reddening area is controlled daily with respect to control animals with no application, and immediately afterwards the medicament is applied again in the various forms.

On the 5^{th} day the average extension of the erythema surface is measured and its reduction in percentage with respect to the animals with no application is calculated.

The results are listed in the following table:

| Group | % reduction of the erythema extension with respect to controls on the 5^{th} day after the induction |
|---|---|
| Controls-no application | ----- |
| Application of normal gauze | 0.0 |
| Application of gauze with immunostimulator (example I.F. above) comparative | 65.0 |
| Application of gauze with hydrocortisone (example III.A. above) comparative | 60.0 |
| Application of gauze derivatized through use of poly linker: Cotton-S-S-cysteyl-polylysyl-(PABA-hydrocortisone)ₙ, the poly lysyl residue .having a MW of 1000-4000 invention | 95% |
| Application of gel with hydrocortisone | 25.0 |

These results point out a significant acceleration in the regression of the erythematic process with the application of sanitary gauzes (i.e. derivatized gauzes), which is even higher than the drug used in form of gel.

Some animals were exposed to UV rays for a prolonged time until actual tissue sores formed on their skin.

### 8. Performance of anti-mite fabrics.

Cotton fibres conjugated to acaricides (as set out in the chemical experimental part above) were used for obtaining fabrics (woven and non-woven) used in turn for the manufacture of household articles (curtains, carpet floor and the like) placed in living environments. In particular, living environments (flats, apartments and houses) at the countryside at locations particularly exposed to dust and ventilation were chosen. Moreover, the tests were conducted during spring, which is the period with the highest mite contamination risk.

After two days of total exposure of the living environments to the open air, at the point in time zero, the dust was removed through vacuum cleaning and the presence and the status of the mite population contained therein was subjected to microscopic examination. Thereafter, some of the living environments, after appropriate randomization, were equipped with the fabrics according to the invention obtained from fibres conjugated to acaricides, thus making up a first test group. On the other hand, the remaining living environments were equipped with fabrics from customary cotton thereby making up a second test group as a control for the development of the mite population.

After another two days, the dust from both of the test groups was collected and the overall number of mites as well as the number of living mites was established. The results of the first test group, expressed as percentage of decrease with respect to the results obtained with the control group (underivatized fabrics) were established for both, for the overall and for the living population.

| Product according to chemical example | Decrease (%) of total living mite population |
|---|---|
| III.B.2.1.1. comparative | 58 |
| IILB.2.1.2. comparative | 59 |
| III.B.2.2.1. invention | 94 |
| III.B.2.2.2. invention | 100 |

Moreover, the dust was analyzed for the presence of active agent in an attempt to verify whether, under realistic working conditions, release of the acaricide into the surrounding environment takes place. The results are reported in the table herein below.

| Product according to chemical example. | Quantity of acaricide per 100g dust |
|---|---|
| IIIB2.1.1. comparative | Significant traces |
| IIIB2.1-2. comparative | Absent |
| IIIB2.2.1. invention | Traces |
| IIIB2.2.2. invention | Absent |

It is noted that the acaracides, in view of their irritating properties, should not come into contact with human skin or be inhaled. It is therefore important that the acaricides are not released into the environment.

To establish the residual risk arising from exposure, the following experimental protocol was followed:
(1) Washing with surfactants (detergents) of common use within normal household practice; the presence of benzyl benzoate within the washing liquors was determined with gas chromatographic equipment.
(2) Exposition to air for a period of six months under normal environmental conditions. The amount of acaricide still bound to the fibre was established thereafter through quantitative hydrolysis and subsequent analysis of the hydrolysate with the same equipment as in (1).

| Product according to chemical example | (1) Percentage of acaricide released into the washing liquor after washing the residual acaricide still with surfactants | (2) Percentage of acaricide released after exposure to air for 6 months (determined through measurement of the residual acaricide still present on the fibre) |
|---|---|---|
| IIIB2.1.1. comparative | 60,0 | 42,0 |
| IIIB2.1.2. comparative | 18,0 | 14,0 |
| IIIB2.2.1. invention | 28,0. | 22,0 |
| IIB2.2.2. invention | 2,0 | 0 |

### TESTS FOR OPTIMIZATION OF THE CONJUGATION BETWEEN POLYSACCHARIDE POLYMERS AND A GIVEN PHRAMACOLOGICALLY OR BIOLOGICALLY ACTIVE SUBSTANCE

### Experiment 1

The following table 1 shows some examples of the actually achievable load with pharmacologically or biologically active substance obtainable by the present invention. The load has been expressed in terms of milligrams of active substance per grams of Polymer (cellulose). In examples (A)-(D), the polymer had been previously activated through controlled oxidation with sodium metaperiodate, and the activated polymer was then conjugated directly (where possible) or through suitable linkers or polylinkers according the invention to the active substance, In examples (E)-(I), the polymer had been previously activated to give the thiolated polymer (reagent W as described in the preamble to chemical experimental section ll above) and the activated polymer was then conjugated directly (where possible) or through suitable linkers or instead polylinkers according the invention to the active substance.

**Table 1:**

| | | | |
|---|---|---|---|
| active substance | Load achievable through direct conjugation [mg/g] conjugation | Load achievable through conjugation through linker [mg/g] | Load achievable through conjugation through polylinker [mg/g] |
| (A) 4-hydroxy benzaldehyde | Not tested | Linker: lysine (as example, IB2 above) 38,0 comparative | Linker: polylysine (MW 1000=4000) 144,0 invention |
| (B) sulfadiazine | (as example IC2 above) 64,0 comparative | Linker: lysine 97,0 comparative | Linker. polylysine (MW 1000-4000) 192,0 invention |
| (C) procaine | (as example ID above) 17,0 comparative | Linker: lysine 28,0 comparative | Linker: polylysine (MW 1000-4000) 116,0 invention |
| (D) Fibrinogen | 1,7 comparative | Linker: lysine (as example 2 above) 10,0 comparative | Linker: polylysine IE(MW 1000-4000) 34,0 invention |
| (E) IgG | (as example IIG above) 2,2 comparative | Linker: lysine, (IgG oxidized to form a Schiff base with the lysyl linker) 6,7 comparative | Linker: poly lysine (MW 1000-4000), IgG oxidized to form a Schiff base with the lysyl linker 96,0 invention |
| (F) hyaluronic acid | Not tested | Linker: S-cysteine (as , example IIH1 above) 9,0 comparative | Linker: S-cysteyl-polylysine (MW 1000-4000) 84,9 invention |
| (H) collagenase | Not tested | Linker: S-cysteine. (as example II. 1 above) 57,0 comparative | Linker: S-cysteyl-polylisine (MW 1000-4000) 101,0 invention |
| (I) lipase | Not | tested Linker: S-cysteine (as example If F above) 52,0 comparative | Linker: S-cysteyl-polylisine (MW 1000-4000) 104,4 invention |

Examples (A)-(I) as above show that using poly linkers, a considerable increase of load can be obtained. Importantly, such increase is achievable without detrimentally affecting the mechanical properties of the polymer substrate as would be the case if the polymer's activation was conducted under more harsh conditions. It is further important to realize that, depending to the combination of linker and active substance involved, the increase of load provided through the use of the inventive poly linkers may arise not only in case of poly valent poly linkers, i.e. if, depending on the type of linkage between polylinker .and active substance, a "leverage effect" with respect to the number of active sites initially created on the polymer becomes available, but also in case of mere linker extension ("spacer effect"), not taking benefit from the leverage effect.

## Claims

1. Conjugate comprising a polysaccharide polymer of natural origin chemically bound to a pharmacologically or biologically active substance through a polylinker.

2. Conjugate according to claim 1 in which said polylinker comprises a polylylsyl residue or polyglutamic acid residue.

3. Conjugate according to claim 1 or 2, in which said polysaccharide is of vegetal origin.

4. Conjugate according to claim 3, in which said polysaccharide consists of units of poly-D-glucose.

5. Conjugate according to claim 4, **characterized in that** said units of D-glucose are bound one to the other so as to form cellulose.

6. Conjugate according to claim 5 **characterized in that** said cellulose forms macroscopic fibres, in particular cotton or viscose fibres.

7. Conjugate according to claim 1 to 6 conjugated with a pharmacologically or biologically active substance having anti-bacterial, anti-inflammatory, antibiotic, mycostatic, anti-mycotic, disinfectant, anti-hemorrhagic, antiseptic, immunostimulating, anti-traumatic, anti-allergic, cicatrizing, riepithelializing, anti-erythemic, anti-dermatopathic, anti-bum, anesthetic, anti-coagulating, coagulating, anti-varix, anti-phlebitic, anti-aggregating for platelets, fibrinolytic, lipolytic, wrinkle-preventing, anti-mite or anti-aging properties.

8. Conjugate according to claims 1 to 6 in which the pharmacologically or biologically active substance is an acaricide.

9. Conjugate according to claim 8 in which said acaricide is benzylbenzoate.

10. Conjugate according to claims 1 to 7, in which said polylinker comprises a polylysyl residue having a molecular weight of less than 16000.

11. Conjugate according to claim 10, in which said polylysyl residue has a molecular weight comprised between 1000 and 4000.

12. Conjugate according to claim 10 or 11 in which said polylinker comprises a cysteinyl polylysyl residue.

13. Conjugate according to claims 10 to 12 in which the pharmacologically or biologically active substance is an anti-inflammatory agent.

14. Conjugate according to claim 13 in which said antiinflammatory agent is naproxen.

15. Yarns or non-woven fabric consisting of fibres of at least a conjugate according to one of the preceding claims.

16. Fabric or non-woven fabric consisting of at least a yarn according to claim 15.

17. Use of at least a conjugate according to one of the claims 1-14 or use of a yarn, fabric or non-woven fabric according to claim 15 or 16 for the preparation of a medicament.

18. Medicinal items for topical application comprising at least a conjugate according to one of the claims 1-14, or a yarn, fabric or non-woven fabric according to claim 15 or 16.

19. Use of at least a conjugate according to one of the claims 1-14 or use of a yarn, fabric or non-woven fabric according to claim 15 or 16 in the preparation of a drug for the topical therapy of hemorrhages, infections, mycoses, inflammations, traumas, bums, allergies, erythemas, dermatopathies, necroses, urticarias, psoriases, pemphigus.

20. Use of at least a conjugate according to one of the claims 1-14, or use of a yarn, fabric or non-woven fabric according to claim 15 or 16 in the preparation of a medicament for topical application for the induction of cicatrisation, for the immunostimulation or for the prevention of allergies.

21. Use of at least a conjugate according to one of the claims 1-14, or use of a yarn, fabric or non-woven fabric according to claim 15 or 16 for the prevention of skin wrinkles, for the prevention of skin aging processes, to obtain hygienic items or for the preparation of a medicament for the reduction of body fat.

22. Hygienic or cosmetic item comprising a conjugate according to claim 1-14 or a yarn, fabric or non-woven fabric according to claim 15 or 16.

23. Use of at least a conjugate according to one of the claims 1-14, or use of a yarn, fabric or non-woven fabric according to claim 15 or 16 in the field of food industry, for the production of sausages (salamis) or cheese.

24. Food filter or wrapping consisting of a conjugate according to claim 1-14 or of a yarn, fabric or non-woven fabric according to claim 15 or 16.

25. Household article consisting of a conjugate according to claim 1-14 or of a yam, fabric or non-woven fabric according to claim 15 or 16.

26. Method for preparing a conjugate according to one of the claims 1-14, comprising the following steps:
a) the creation, on the fibres of the polysaccharide polymer of sites which are suitable for chemical conjugation, by modifying at least some of the alcohol OH groups which are present on the polysaccharide polymer with a polylinker, thus obtaining an activated polysaccharide polymer, and
b) the reaction between the activated polysaccharide polymer and a pharmacologically or biologically active substance or of a derivative thereof under formation of a chemical conjugation between the activated polysaccharide polymer and the pharmacologically or biologically active substance via said polylinker, and optionally
c) the modification, by means of a chemical reaction, of the nature of the chemical conjugation established in steps (a) and (b) between the polysaccharide polymer and the pharmacologically or biologically active substance.

## Patentansprüche

1. Konjugat, enthaltend ein Polysaccharidpolymer natürlicher Herkunft, das durch einen Polylinker chemisch an eine pharmakologisch oder biologisch aktive Substanz gebunden ist.

2. Konjugat gemäß Anspruch 1, wobei genannter Polylinker einen Polylysylrest oder einen Polyglutaminsäurerest enthält.

3. Konjugat gemäß Anspruch 1 oder 2, wobei genanntes Polysaccharid pflanzlicher Herkunft ist.

4. Konjugat gemäß Anspruch 3, wobei genanntes Polysaccharid aus Poly-D-Glucoseeinheiten besteht.

5. Konjugat gemäß Anspruch 4, **dadurch gekennzeichnet, dass** genannte D-Glucoseeinheiten so aneinander gebunden sind, dass sie Cellulose bilden.

6. Konjugat gemäß Anspruch 5 **dadurch gekennzeichnet, dass** genannte Cellulose makroskopische Fasern bildet, insbesondere Baumwoll- oder Viskosefasern.

7. Konjugat gemäß Anspruch 1 bis 6, konjugiert mit einer pharmakologisch oder biologisch aktiven Substanz mit antibakteriellen, entzündungshemmenden, antibiotischen, fungistatischen, antimykotischen, desinfizierenden, antihämorrhagischen, antiseptischen, immunstimulierenden, antitraumatischen, antiallergischen, vernarbenden, reepithelisierenden, Anti-Erythema-, antidermatopathischen, gegen Verbrennungen gerichteten, betäubenden, antikoagulierenden, koagulierenden, Krampfadern mildernden, antiphlebitischen, gegen Thrombozytenaggregation wirkenden, fibrinolytischen, lipolytischen, faltenverhindernden, anti-Milben- oder Antiaging-Eigenschaften.

8. Konjugate gemäß den Ansprüchen 1 bis 6, wobei die pharmakologisch oder biologisch aktive Substanz ein Akarizid ist.

9. Konjugat gemäß Anspruch 8, wobei das genannte Akarizid ein Benzylbenzoat ist.

10. Konjugat gemäß den Ansprüchen 1 bis 7, wobei genannter Polylinker einen Polylysylrest mit einem Molekulargewicht von weniger als 16000 umfasst.

11. Konjugat gemäß Anspruch 10, wobei genannter Polylysylrest ein Molekulargewicht aufweist, das zwischen 1000 und 4000 beträgt.

12. Konjugat gemäß Anspruch 10 oder 11, wobei der genannte Polylinker einen Cysteinyl-Polylylsylrest enthält.

13. Konjugat gemäß den Ansprüchen 10 bis 12, wobei die pharmakologisch oder biologisch aktive Substanz ein entzündungshemmendes Mittel ist.

14. Konjugat gemäß Anspruch 13, wobei das genannte entzündungshemmende Mittel Naproxen ist.

15. Garne oder Vliesgewebe, bestehend aus Fasern aus mindestens einem Konjugat gemäß einem der vorhergehenden Ansprüche.

16. Gewebe oder Vliesgewebe, bestehend aus mindestens einem Garn gemäß Anspruch 15.

17. Verwendung von mindestens einem Konjugat gemäß einem der Ansprüche 1 - 14 oder Verwendung eines Garns, Gewebes oder Vliesgewebes gemäß Anspruch 15 oder 16 für die Herstellung eines Arzneimittels.

18. Medizinische Artikel für topische Anwendung, umfassend mindestens ein Konjugat gemäß einem der Ansprüche 1-14 oder ein Garn, Gewebe oder Vliesgewebe gemäß Anspruch 15 oder 16.

19. Verwendung von mindestens einem Konjugat gemäß einem der Ansprüche 1-14 oder Verwendung eines Garns, Gewebes oder Vliesgewebes gemäß Anspruch 15 oder 16 bei der Herstellung eines Wirkstoffs für die topische Therapie von Blutungen, Infektionen, Mykosen, Entzündungen, Traumata, Verbrennungen, Allergien, Erythema, Hauterkrankungen, Nekrosen, Urtikarien, Psoriasis, Pemphigus.

20. Verwendung von mindestens einem Konjugat gemäß einem der Ansprüche 1 - 14 oder Verwendung eines Garns, Gewebes oder Vliesgewebes gemäß Anspruch 15 oder 16 bei der Herstellung eines Arzneimittels für die topische Anwendung zur Induktion einer Vernarbung, für die Immunstimulierung oder für die Vorbeugung von Allergien.

21. Verwendung von mindestens einem Konjugat gemäß einem der Ansprüche 1 - 14 oder Verwendung eines Garns, Gewebes oder Vliesgewebes gemäß Anspruch 15 oder 16 für die Vorbeugung von Hautfalten, für die Verhinderung des Hautalterungsprozesse, zum Erhalt von hygienischen Artikeln oder für die Herstellung eines Arzneimittels zur Verminderung des Körperfetts.

22. Hygienische oder kosmetische Artikel, enthaltend mindestens ein Konjugat gemäß einem der Ansprüche 1 - 14 oder ein Garn, Gewebe oder Vliesgewebe gemäß Anspruch 15 oder 16.

23. Verwendung von mindestens einem Konjugat gemäß einem der Ansprüche 1-14 oder Verwendung eines Garns, Gewebes oder Vliesgewebes gemäß Anspruch 15 oder 16 auf dem Gebiet der Nahrungsmittelindustrie für die Herstellung von Würsten (Salamis) oder Käse.

24. Nahrungsfilter oder -verpackungen, bestehend aus einem Konjugat gemäß einem der Ansprüche 1 - 14 oder einem Garn, Gewebe oder Vliesgewebe gemäß Anspruch 15 oder 16.

25. Haushaltsartikel, bestehend aus einem Konjugat gemäß einem der Ansprüche 1 - 14 oder einem Garn, Gewebe oder Vliesgewebe gemäß Anspruch 15 oder 16.

26. Verfahren zur Herstellung eines Konjugats gemäß einem der Ansprüche 1 - 14, umfassend die folgenden Schritte:
a) die Bildung, auf den Fasern eines Polysaccharidpolymers, von Stellen, die für chemische Konjugation geeignet sind, durch Modifizieren von mindestens einigen der Alkohol-OH-Gruppen, die auf dem Polysaccharidpolymer vorkommen, mit einem Polylinker, wodurch ein aktiviertes Polysaccharidpolymer erhalten wird, und
b) die Reaktion zwischen dem aktivierten Polysaccharidpolymer und einer pharmakologisch oder biologisch aktiven Substanz oder eines Derivats davon unter Bildung einer chemischen Konjugation zwischen dem aktivierten Polysaccharidpolymer und der pharmakologisch oder biologisch aktiven Verbindung über den genannten Polylinker und gegebenenfalls
c) die Modifizierung, mittels einer chemischen Reaktion, der Art der chemischen Konjugation, die in den Schritten (a) und (b) zwischen dem Polysaccharidpolymer und der pharmakologisch oder biologisch aktiven Substanz eingeführt wurde.

## Revendications

1. Conjugué comprenant un polymère de polysaccharide d'origine naturelle chimiquement lié à une substance pharmacologiquement ou biologiquement active par l'intermédiaire d'un lieur multisite.

2. Conjugué selon la revendication 1 dans lequel ledit lieur multisite comprend un résidu polylylsyle ou un résidu acide glutamique.

3. Conjugué selon la revendication 1 ou 2, dans lequel ledit polysaccharide est d'origine végétale.

4. Conjugué selon la revendication 3, dans lequel ledit polysaccharide est constitué d'unités de poly-D-glucose.

5. Conjugué selon la revendication 4, **caractérisé en ce que** lesdites unités de D-glucose sont liées les unes aux autres de manière à former de la cellulose.

6. Conjugué selon la revendication 5 **caractérisé en ce que** ladite cellulose forme des fibres macroscopiques, en particulier des fibres de coton ou de viscose.

7. Conjugué selon les revendications 1 à 6 conjugué avec une substance pharmacologiquement ou biologiquement active ayant des propriétés antibactérienne, anti-inflammatoire, antibiotique, mycostatique, antifongique, désinfectante, antihémorragique, antiseptique, immuno-stimulante, antitraumatique, antiallergique, cicatrisante, épithélialisante, antiérythème, antidermopathique, anti-brûlure, anesthésique, anticoagulante, coagulante, anti-varice, antiphlébite, antiaggrégation pour les plaquettes, fibrinolytique, lipolytique, de prévention des rides, antimite ou antivieillissement.

8. Conjugué selon les revendications 1 à 6 dans lequel la substance pharmacologiquement ou biologiquement active est un acaricide.

9. Conjugué selon la revendication 8 dans lequel ledit acaricide est le benzoate de benzyle.

10. Conjugué selon les revendications 1 à 7, dans lequel ledit lieur multisite comprend un résidu polylylsyle ayant un poids moléculaire de moins de 16000.

11. Conjugué selon la revendication 10, dans lequel ledit résidu polylylsyle a un poids moléculaire compris entre 1000 et 4000.

12. Conjugué selon la revendication 10 ou 11 dans lequel ledit lieur multisite comprend un résidu cystéinyl polylylsyle.

13. Conjugué selon les revendications 10 à 12 dans lequel la substance pharmacologiquement ou biologiquement active est un agent anti-inflammatoire.

14. Conjugué selon la revendication 13 dans lequel ledit agent anti-inflammatoire est le naproxène.

15. Fils de textile non-tissé constitués de fibres d'au moins un conjugué selon l'une des revendications précédentes.

16. Textile ou textile non-tissé constitué d'au moins un fil selon la revendication 15.

17. Utilisation d'au moins un conjugué selon l'une des revendications 1 à 14 ou utilisation d'un fil, textile ou textile non-tissé selon la revendication 15 ou 16 pour la préparation d'un médicament.

18. Articles médicinaux pour application locale comprenant au moins un conjugué selon l'une des revendications 1 à 14, ou un fil, textile ou textile non-tissé selon la revendication 15 ou 16.

19. Utilisation d'au moins un conjugué selon l'une des revendications 1 à 14 ou utilisation d'un fil, textile ou textile non-tissé selon la revendication 15 ou 16 dans la préparation d'un médicament pour la thérapie locale d'hémorragies, d'infections, de mycoses, d'inflammations, de traumatismes, de brulures, d'allergies, d'érythèmes, de dermatopathies, de nécroses, d'urticaires, de psoriasis, de pemphigus.

20. Utilisation d'au moins un conjugué selon l'une des revendications 1 à 14, ou utilisation d'un fil, textile ou textile non-tissé selon la revendication 15 ou 16 dans la préparation d'un médicament pour application locale pour l'induction de cicatrisation, pour l'immunostimulation ou pour la prévention d'allergies.

21. Utilisation d'au moins un conjugué selon l'une des revendications 1 à 14, ou utilisation d'un fil, textile ou textile non-tissé selon la revendication 15 ou 16 pour la prévention de rides de la peau, pour la prévention de processus de vieillissement de la peau, pour obtenir des articles hygiéniques ou pour la préparation d'un médicament pour la réduction des corps gras.

22. Article hygiénique ou cosmétique comprenant un conjugué selon les revendications 1 à 14 ou un fil, textile ou textile non-tissé selon la revendication 15 ou 16.

23. Utilisation d'au moins un conjugué selon l'une des revendications 1 à 14, ou utilisation d'un fil, textile ou textile non-tissé selon la revendication 15 ou 16 dans le domaine de l'industrie alimentaire, pour la production de saucisses (salamis) ou de fromage.

24. Filtre ou enveloppe d'aliments constitué d'un conjugué selon les revendications 1 à 14 ou d'un fil, textile ou textile non-tissé selon la revendication 15 ou 16.

25. Article ménager constitué d'un conjugué selon les revendications 1 à 14 ou d'un fil, textile ou textile non-tissé selon la revendication 15 ou 16.

26. Procédé pour préparer un conjugué selon l'une des revendications 1 à 14, comprenant les étapes suivantes:
a) la création, sur les fibres du polymère de polysaccharide de sites qui sont adaptés pour conjugaison chimique, en modifiant au moins certains des groupes OH d'alcool qui sont présents sur le polymère de polysaccharide avec un lieur multisite de manière à obtenir un polymère de polysaccharide activé, et
b) la réaction entre le polymère de polysaccharide activé et une substance pharmacologiquement ou biologiquement active ou d'un dérivé de celle-ci pour la formation d'une conjugaison chimique entre le polymère de polysaccharide activé et la substance pharmacologiquement ou biologiquement active par l'intermédiaire dudit lieur multisite, et facultativement
c) la modification, au moyen d'une réaction chimique, de la nature de la conjugaison chimique formée dans les étapes (a) et (b) entre le polymère de polysaccharide et la substance pharmacologiquement ou biologiquement active.
